# EUROPEAN PATENT APPLICATION

(11) **EP 4 223 884 A1**
(43) Date of publication of application: **09.08.2023**
(21) Application number: 21875715.1
(22) Date of filing: 29.09.2021
(51) Int. Cl.: C12Q 1/00, C12Q 1/32, C12N 9/04

(54) **METHOD FOR IMPROVING STABILITY OF COMPOSITION CONTAINING FLAVIN-DEPENDENT LACTATE DEHYDROGENASE**

(30) Priority: 29.09.2020 JP 2020163631
(71) Applicant: Kikkoman Corporation, Noda-shi, Chiba 278-8601 (JP)
(72) Inventor: SUZUKI, Ryota, Noda-shi, Chiba 278-8601 (JP); MASAKARI, Yosuke, Noda-shi, Chiba 278-8601 (JP)
(74) Representative: J A Kemp LLP
(86) International application number: PCT/JP2021/035929
(87) International publication number: WO 2022/071417

(57) **Abstract**

Provided is a method for improving thermal stability of a composition comprising flavin-dependent lactate dehydrogenase (LDH). The composition comprising LDH is allowed to coexist with one or more of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; a nonionic polymer; and an ionic polymer. It is possible to reduce deactivation of LDH during preparation and storage of a lactate measuring reagent, a lactate assay kit, a lactate sensor and fuel cell, reduce the amount of LDH to be used; as well as improve thermal stability, storage stability and measurement accuracy of the measuring reagent, assay kit and sensor.

## Description

### Technical Field

The present disclosure relates to a method for improving the stability of a composition comprising a flavin-dependent dehydrogenase and a composition obtained by the method.

### Background Art

Lactate concentrations in the blood and sweat are known as markers reflecting fatigue and physical conditions. Lactate is a major metabolite and serves as an important indicator not only for monitoring health but also for evaluating health including that of patients with serious diseases and/or surgical patients. Lactate value in the body fluid can serve as an indicator for various disease conditions such as circulatory failure and liver damage. Lactate monitoring can be utilized for detecting sepsis, hypoxia, and the existence of cancer tissue (Non Patent Literature 1).

Lactate monitoring is carried out not only for sick people but also for healthy people in order to manage physical conditions and, more specifically, lactate is monitored as an index for monitoring the appropriateness of exercise by athletes and people who are highly interested in daily exercise (Non Patent Literature 2).

As an enzyme that recognizes lactate as a substrate, flavin-dependent lactate dehydrogenase (hereinafter referred to as LDH) is known. Non Patent Literature 3 discloses that known LDHs from *Saccharomyces cerevisiae* have a problem in stability. It should be noted that, while nicotinamide adenine dinucleotide-dependent lactate dehydrogenase is also known as a lactate dehydrogenase, this is not classified as a flavin-dependent lactate dehydrogenase (LDH). As such, the flavin-dependent lactate dehydrogenase (or LDH) referred to in the present specification does not include nicotinamide-dependent lactate dehydrogenase, unless specified otherwise.

### Citation List

### Non Patent Literatures

Non Patent Literature 1: Japanese Sepsis Clinical Practice Guidelines 2016, PNAS September 15, 2015, 112 (37), 11642-11647
Non Patent Literature 2: Sports Performance Research, 3, 31-48, 2011
Non Patent Literature 3: Methods Enzymol., 53, 238-56, 1978

### Summary of Invention

### Technical Problem

An object of the present disclosure is to provide a method for improving the stability of a composition comprising LDH and a sufficiently stable lactate measurement composition obtained by the method. According to a particular embodiment, an object of the present disclosure is to provide a method for suppressing the decrease or deactivation of lactate dehydrogenase (LDH) activity when LDH is applied to an electrode.

### Solution to Problem

To attain the above objects, the present inventors have conducted inventive studies. As a result, the present inventors have found that a composition comprising LDH in the presence of various compounds provides an improved stability of LDH, and that inactivation of LDH when applied to an electrode can be suppressed. The present inventors have completed the present disclosure including these embodiments.

More specifically, the present disclosure provides the following embodiments.
[1] A method for improving thermal stability of a composition comprising lactate dehydrogenase, comprising a step of subjecting a composition comprising lactate dehydrogenase requiring a flavin compound as a coenzyme in the presence of at least one of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; a polyethylene glycol; or an ionic polymer.
[2] The method according to [1], wherein the lactate dehydrogenase is a lactate dehydrogenase from the genus *Saccharomyces,* the genus *Pichia,* the genus *Candida,* the genus *Ogataea,* the genus *Brettanomyces,* the genus *Cyberlindnera,* the genus *Hanseniaspora,* the genus *Kazachstania,* the genus *Kluyveromyces,* the genus *Lachancea,* the genus *Naumovozyma,* the genus *Tetrapisispora,* the genus *Torulaspora,* the genus *Vanderwaltozyma,* the genus *Zygosaccharomyces,* the genus *Zygotorulaspora,* the genus *Myceliophthora,* the genus *Thermothelomyces*, the genus *Chaetomium or* the genus *Madurella.*
[3] The method for improving thermal stability of a composition comprising lactate dehydrogenase according to [1], wherein the lactate dehydrogenase is the following (i) and/or (ii):
   (i) lactate dehydrogenase comprising an amino acid sequence, which has an identity of 70% or more or 80% or more to the amino acid sequence of SEQ ID NO: 1, SEQ ID NO: 4, SEQ ID NO: 7 or SEQ ID NO: 10,
   (ii) lactate dehydrogenase, characterized in that the amino acid sequence of a homologous region consisting of amino acid sequences at positions 138 to 140, positions 186 to 194, positions 217 to 222, positions 243 to 245, positions 271 to 278, positions 280 to 283, positions 355 to 367, positions 398 to 401, positions 403 to 406 and positions 425 to 433 in SEQ ID NO: 4 has a sequence identity of 80% or more or 90% or more to the amino acid sequence of a homologous region of the corresponding positions in the lactate dehydrogenase.
[4] The method for improving thermal stability of a composition comprising lactate dehydrogenase according to any one of [1] to [3], wherein
   the monocarboxylic acid is selected from the group consisting of gluconic acid, leucine acid, 3-phenyllactic acid, glycine, lysine, histidine and arginine,
   the dicarboxylic acid is selected from the group consisting of malic acid, succinic acid, maleic acid, fumaric acid, malonic acid, methylmalonic acid, oxaloacetic acid, tartaric acid, oxalic acid, succinic acid, glutaric acid, α-ketoglutaric acid, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid and 3-hydroxyasparagine,
   the tricarboxylic acid is selected from the group consisting of citric acid, isocitric acid, aconitic acid, trimesic acid, 1,2,3-propanetricarboxylic acid and 2-phosphonobutane-1,2,4-tricarboxylic acid,
   the tetracarboxylic acid is selected from ethylenediaminetetraacetic acid,
   the phosphoric acid is selected from the group consisting of a phosphate and a pyrophosphate,
   the sulfuric acid is selected from a sulfate,
   the monosaccharide is selected from the group consisting of myo-inositol,
   the disaccharide is selected from the group consisting of sucrose, trehalose and maltose, or
   polyethylene glycol is used,
      or
   the ionic polymer is an anionic polymer or a cationic polymer,
   the anionic polymer is selected from the group consisting of a polyacrylic acid, hyaluronic acid and carboxymethyl cellulose or a salt thereof, or
   the cationic polymer is selected from polylysine, polyethylene imine, methyl glycol chitosan and poly(diallyldimethylammonium chloride).
[5] The method for improving thermal stability of a composition comprising lactate dehydrogenase according to any one of [1] to [4], wherein the anion, which is selected from the group consisting of a monocarboxylic acid, a dicarboxylic acid, a tricarboxylic acid, a tetracarboxylic acid, a polycarboxylic acid, phosphoric acid, sulfuric acid or a salt thereof, the monosaccharide, disaccharide, anionic polymer or cationic polymer is included in a solution within a concentration range of from 0.01 wt% to 30 wt%.
[6] A composition for measuring lactate comprising: a lactate dehydrogenase requiring a flavin compound as a coenzyme; and at least one compound of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; polyethylene glycol; and an ionic polymer.
[7] The composition for measuring lactate according to [6], wherein the lactate dehydrogenase is a lactate dehydrogenase from the genus *Saccharomyces,* the genus *Pichia,* the genus *Candida,* the genus *Ogataea,* the genus *Brettanomyces,* the genus *Cyberlindnera,* the genus *Hanseniaspora,* the genus *Kazachstania,* the genus *Kluyveromyces,* the genus *Lachancea,* the genus *Naumovozyma,* the genus *Tetrapisispora,* the genus *Torulaspora,* the genus *Vanderwaltozyma,* the genus *Zygosaccharomyces,* the genus *Zygotorulaspora,* the genus *Myceliophthora,* the genus *Thermothelomyces*, the genus *Chaetomium or* the genus *Madurella.*
[8] The composition according to [6], wherein the lactate dehydrogenase is the following (i) and/or (ii),
   (i) lactate dehydrogenase comprising an amino acid sequence, which has an identity of 70% or more or 80% or more to the amino acid sequence of SEQ ID NO: 1, SEQ ID NO: 4, SEQ ID NO: 7 or SEQ ID NO: 10,
   (ii) lactate dehydrogenase, characterized in that the amino acid sequence of a homologous region consisting of amino acid sequences at positions 138 to 140, positions 186 to 194, positions 217 to 222, positions 243 to 245, positions 271 to 278, positions 280 to 283, positions 355 to 367, positions 398 to 401, positions 403 to 406 and positions 425 to 433 in SEQ ID NO: 4 has a sequence identity of 80% or more or 90% or more to the amino acid sequence of a homologous region of the corresponding positions in the lactate dehydrogenase.
[9] The composition for measuring lactate according to any one of [6] to [8], wherein
   the monocarboxylic acid is selected from the group consisting of gluconic acid, leucine acid, 3-phenyllactic acid, glycine, lysine, histidine and arginine,
   the dicarboxylic acid is selected from the group consisting of malic acid, succinic acid, maleic acid, fumaric acid, malonic acid, methylmalonic acid, oxaloacetic acid, tartaric acid, oxalic acid, succinic acid, glutaric acid, α-ketoglutarate, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid and 3-hydroxyasparagine,
   the tricarboxylic acid is selected from the group consisting of citric acid, isocitric acid, aconitic acid, trimesic acid, 1,2,3-propanetricarboxylic acid and 2-phosphonobutane-1,2,4-tricarboxylic acid,
   the tetracarboxylic acid is selected from ethylenediaminetetraacetic acid,
   the phosphoric acid is selected from the group consisting of a phosphate and a pyrophosphate,
   the sulfuric acid is selected from a sulfate,
   the monosaccharide is selected from the group consisting of myo-inositol,
   the disaccharide is selected from the group consisting of sucrose, trehalose and maltose or
   polyethylene glycol is used,
      or
   the ionic polymer is an anionic polymer or a cationic polymer,
   the anionic polymer being selected from the group consisting of a polyacrylic acid, hyaluronic acid and carboxymethyl cellulose or a salt thereof, or
   the cationic polymer being selected from polylysine, polyethylene imine, methyl glycol chitosan and poly(diallyldimethylammonium chloride).
[10] The composition for measuring lactate according to any one of [6] to [9], wherein the anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; a polyethylene glycol; an anionic polymer; or a cationic polymer is included in a solution within a concentration range of from 0.01 wt% to 30 wt%.
[11] A kit for measuring lactate comprising the compound according to [6] to [10].
[12] An electrode comprising a composition comprising: lactate dehydrogenase requiring a flavin compound as a coenzyme; and at least one compound of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; polyethylene glycol; and an ionic polymer.
[13] A lactate sensor comprising the electrode according to [12].
[14] A fuel cell comprising the electrode according to [12].
[15] A method of suppressing the decrease or deactivation of lactate dehydrogenase activity in a method of producing an electrode comprising a step of applying lactate dehydrogenase to an electrode, said method comprising the steps of:
   i) providing a solution comprising lactate dehydrogenase,
   ii) adding, to the solution, at least one stabilizer selected from the group consisting of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; polyethylene glycol; and an ionic polymer,
   iii) applying the solution comprising lactate dehydrogenase and the stabilizer to an electrode, and
   iv) drying the solution applied.
[16] The production method according to [15], wherein the electrode to which lactate dehydrogenase is applied is comprised in a lactate sensor.

The present specification encompasses the contents disclosed in JP Patent Application No. 2020-163631, based on which the priority was claimed.

### Advantageous Effects of Invention

Due to the present disclosure it is possible to reduce deactivation of LDH during production and storage of a lactate measuring reagent, a lactate assay kit and a lactate sensor, thereby reducing the amount of LDH required for producing the reagent, kit and sensor, and improving storage stability and measurement accuracy of final products of the reagent, kit and sensor.

### Brief Description of Drawing

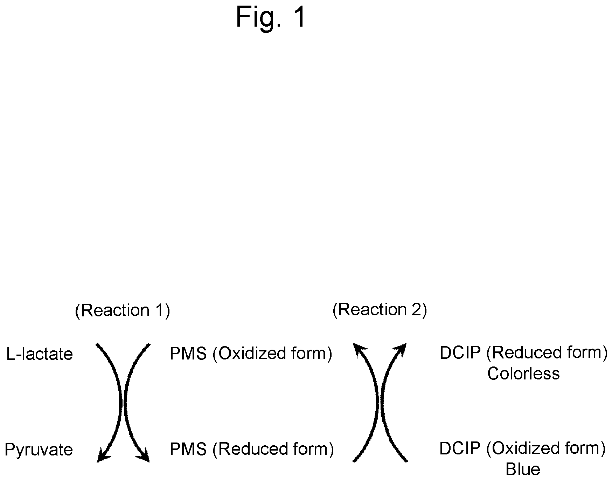

[Figure 1] The figure shows a scheme of dehydrogenase reaction by lactate dehydrogenase.

### Description of Embodiments

The present disclosure is characterized in that thermal stability of a composition comprising LDH can be improved by allowing any LDH to coexist with an additive ingredient. Attempts to improve the thermal stability of LDH by modifying the LDH *per se* are not known. Further, findings of thermal stability improving agents effective on LDH and compositions comprising LDH having improved thermal stability were scarce. In one embodiment, the present disclosure provides a method for improving the stability of a composition comprising LDH. As a compound to be added for improving stability of the composition comprising LDH of the present disclosure, an anion, a sugar or an ionic polymer was found to be effective. More specifically, in one embodiment, the present disclosure provides a stabilizer for improving the stability of a composition comprising LDH. The stabilizer for improving the stability of a composition comprising LDH may comprise an anion, a sugar and/or an ionic (cationic, anionic, zwitterionic) polymer.

The anion according to the present disclosure is not particularly limited as long as it improves the thermal stability of a composition comprising LDH of the present disclosure and, examples of the anion include a compound comprising a carboxyl group, a halogen compound, a phosphate, a nitrate, a sulfate, a protein, a peptide and an amino acid. Examples of the compound comprising a carboxyl group include, but are not limited to, a monocarboxylic acid, a dicarboxylic acid, a tricarboxylic acid, a tetracarboxylic acid and a polycarboxylic acid compound. In particular, the anion to be preferably used in the present invention is at least one type of dicarboxylic acid or tricarboxylic acid, tetracarboxylic acid, polycarboxylic acid or a salt thereof, but is not limited to the same. Examples of a nonionic polymer may include polyethylene glycol. Examples of the ionic polymer may include a cationic polymer, an anionic polymer or a zwitterionic polymer. Examples of the cationic polymer include, but are not limited to, polylysine, polyethylene imine, methyl glycol chitosan and poly(diallyldimethylammonium chloride). Examples of the anionic polymer include, but are not limited to, hyaluronic acid, a cellulose derivative (e.g., carboxymethyl cellulose), a polyacrylic acid derivative (e.g., polyacrylic acid)) and a polymethacrylic acid derivative (e.g., polymethyl methacrylate and polyhydroxymethyl methacrylate). Examples of the zwitterionic polymer include, but are not limited to, poly(carboxybetaine methacrylate) disclosed, for example, in JP Patent Publication (Kokai) No. 2017-527669 A. In specific embodiments, the compound to be added for improving the stability of a composition comprising LDH is specifically referred to as an LDH stabilizer or simply, a stabilizer. However, this does not prevent the LDH stabilizer from comprising other compounds. More specifically, in another embodiment, the stabilizer to be added to improve the stability of a composition comprising LDH comprises a compound that stabilizes LDH. Further, the stabilizer may comprise any other compound. With regard to the composition of the present disclosure, according to a specific embodiment, ammonium sulfate, and, in particular, 50 wt% or more of ammonium sulfate, is excluded from the sulfate.

In one embodiment, the monocarboxylic acid to be used in the present disclosure is not particularly limited as long as it improves stability of the composition comprising LDH of the present disclosure and preferred examples of the monocarboxylic acid include gluconic acid, leucine acid, 3-phenyllactic acid, glycine, arginine, lysine and histidine. In one embodiment, the monocarboxylic acid to be used in the present disclosure, may have 7 or less carbon atoms in the main chain. In one embodiment, preferred examples of the dicarboxylic acid to be used in the present disclosure include malic acid, succinic acid, maleic acid, fumaric acid, malonic acid, methylmalonic acid, oxaloacetic acid, tartaric acid, oxalic acid, succinic acid, glutaric acid, α-ketoglutarate, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid and 3-hydroxyaspartic acid and the like. In one embodiment, preferred examples of the tricarboxylic acid to be used in the present disclosure include citric acid, isocitric acid, aconitic acid, trimesic acid, 1,2,3-propanetricarboxylic acid and 2-phosphonobutane-1,2,4-tricarboxylic acid and the like. In one embodiment, preferred examples of the tetracarboxylic acid to be used in the present disclosure include ethylenediaminetetraacetic acid (EDTA) and the like. In one embodiment, the sugar to be used in the present disclosure is not particularly limited as long as it improves stability of the composition comprising LDH of the present disclosure and include, for example, a monosaccharide, a disaccharide and an oligosaccharide and the like. Preferred examples of the sugar include sucrose, trehalose, maltose and myo-inositol and the like. In one embodiment, the molecular weight of a preferable polyethylene glycol to be used in the present disclosure is not particularly limited and, for example, a preferred average molecular weight thereof is 300 to 100,000, particularly 7,300 to 9,300. In one embodiment, preferred examples of the cationic polymer to be used in the present disclosure include polylysine, polyethylene imine, methyl glycol chitosan and poly(diallyldimethylammonium chloride). Examples of polylysine include α-polylysine and ε-polylysine. The molecular weight of the polylysine is not particularly limited and, for example, the average molecular weight thereof is preferably 300 to 100,000 and further preferably 3,500 to 6,000 (e.g., 3,000 to 5,000 or 3,500 to 4,500 but not limited thereto). A polyethylene imine may be a straight or branched type. The molecular weight of the polyethylene imine is not particularly limited and an average molecular weight of polyethylene imine that can be used herein is, for example, 300 to 100,000 (e.g., 3000 to 20,000, e.g., 3500 to 10,000). In one embodiment, preferred examples of the anionic polymer to be used in the present disclosure include polyacrylic acid, polystyrene sulfonic acid, hyaluronic acid or a salt thereof and carboxymethyl cellulose or a salt thereof. The molecular weight of the polyacrylic acid is not particularly limited. Preferred examples of an average molecular weight of the polyacrylic acid include, but are not limited to, 1000 to 1,250,000. The molecular weight of the polystyrene sulfonic acid is not particularly limited and, preferred examples of an average molecular weight of the polystyrene sulfonic acid include, but are not limited to, 1000 to 1,000,000. In one embodiment, preferred examples of the nitrate to be used in the present disclosure include sodium nitrate, ammonium nitrate, magnesium nitrate, potassium nitrate, calcium nitrate and lithium nitrate and the like. In one embodiment, preferred examples of the sulfate to be used in the present disclosure include sodium sulfate, ammonium sulfate, magnesium sulfate, potassium sulfate, calcium sulfate and lithium sulfate and the like. In one embodiment, preferred examples of the phosphate to be used in the present disclosure include sodium phosphate, ammonium phosphate, magnesium phosphate, potassium phosphate, calcium phosphate and lithium phosphate and the like. In one embodiment, preferred examples of the anion or sugar to be used in the present disclosure include a compound comprising at least one carboxyl group, a halogen compound, a phosphate, a nitrate, a sulfate, a protein, a peptide, an amino acid or a salt thereof, or a sugar.

In another embodiment, at least one compound selected from the group consisting of gluconic acid, leucine acid, 3-phenyllactic acid, glycine, arginine, lysine, histidine, malic acid, succinic acid, maleic acid, fumaric acid, malonic acid, methylmalonic acid, oxaloacetic acid, tartaric acid, oxalic acid, succinic acid, glutaric acid, α-ketoglutarate, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid, 3-hydroxyaspartic acid, citric acid, isocitric acid, aconitic acid, trimesic acid, 1,2,3-propanetricarboxylic acid, 2-phosphonobutane-1,2,4-tricarboxylic acid, ethylenediaminetetraacetic acid (EDTA), hyaluronic acid or a salt thereof, carboxymethyl cellulose or a salt thereof, sucrose, trehalose, maltose, myo-inositol, polyethylene glycol, polylysine, α-polylysine, ε-polylysine, polyethylene imine, methyl glycol chitosan and poly(diallyldimethylammonium chloride), polyacrylic acid or a salt thereof, polystyrene sulfonic acid or a salt thereof, sodium nitrate, ammonium nitrate, magnesium nitrate, potassium nitrate, calcium nitrate, lithium nitrate, sodium sulfate, ammonium sulfate, magnesium sulfate, potassium sulfate, calcium sulfate, lithium sulfate, sodium phosphate, ammonium phosphate, magnesium phosphate, potassium phosphate, calcium phosphate, lithium phosphate, a compound comprising at least one carboxyl group, a halogen compound, a phosphate, a nitrate, a sulfate, a protein, a peptide, and an amino acid or a salt thereof may be excluded from the stabilizer of the present disclosure.

If the stabilizer of the present disclosure comprises leucine acid, the leucine acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-leucine acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises 3-phenyllactic acid, the 3-phenyllactic acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-3-phenyllactic acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises arginine, the arginine may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-arginine may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises lysine, the lysine may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-lysine may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises histidine, the histidine may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-histidine may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises malic acid, the malic acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-malic acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises tartaric acid, the tartaric acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-tartaric acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises glutamic acid, the glutamic acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-glutamic acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises aspartic acid, the aspartic acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-aspartic acid may have an optical purity of 90 to 100%ee, e.g., 95 to 100%ee or 97 to 100%ee. It should be noted that, in the present specification, a racemic form is expressed by "DL-" attached before the name of a compound.

If the stabilizer of the present disclosure comprises 3-hydroxyaspartic acid, the 3-hydroxyaspartic acid may be provided as L-form or D-form or a mixture of L-form and D-form, for example, a racemic form. In the present specification, the L-form of an optical active compound refers to a compound in which L-form is present at an optical purity of, for example, 90 to 100%ee, e.g., 95 to 100%ee or 97 to 100%ee. For example, L-3-hydroxyaspartic acid may have an optical purity of 90 to 100%ee, e.g., 95 to 100%ee or 97 to 100%ee. It should be noted that, in the present specification, a racemic form is expressed by "DL-" attached before the name of a compound.

The stabilizer for improving stability of the composition comprising LDH of the present disclosure may be added to a lactate measuring reagent or a lactate measurement composition such that the final concentration of the compound (active ingredient) in the composition comprising LDH becomes 0.1 mM to 1000 mM, e.g., 0.1 mM to 900 mM, 0.1 mM to 800 mM, 0.1 mM to 700 mM, 0.1 mM to 600 mM, 0.1 mM to 500 mM, 0.1 mM to 400 mM, 0.1 mM to 300 mM, 0.1 mM to 200 mM, 0.1 mM to 100 mM, 0.1 mM to 90 mM, 0.1 mM to 80 mM, 0.1 mM to 70 mM, 0.1 mM to 60 mM, 0.1 mM to 50 mM, 0.1 mM to 40 mM, 0.1 mM to 30 mM, 0.2 mM to 20 mM, 0.25 mM to 10 mM, or, for example, 300 mM. In one embodiment, an aqueous solution comprising a stabilizer of the present disclosure to be added for improving stability and LDH can be applied to an electrode and dried. In this case, it is envisaged that the stabilizer (which may be comprised in the composition) is enriched to a higher concentration than the final concentration before drying. Such LDH stabilizer present at a high concentration after dry and the electrode having the stabilizer applied thereon are also encompassed by the present disclosure. Further, an electrode comprising a composition comprising a stabilizer is provided by the present disclosure. Further, an embodiment in which a composition comprising a stabilizer is applied to an electrode, is also provided. In one embodiment, the stabilizer of the present disclosure to be added for improving stability can be added at any time during a production step of LDH. For example, the stabilizer may be added in an early stage of a purification step, in the middle of an extraction/purification step, in a latter half of the production, process, for example, in a stage of lyophilization, powdering, or adjustment. In the present disclosure, other various components required for production of a composition comprising LDH, such as a buffer, a surfactant, a stabilizer and an excipient may also be present in the production of the composition comprising LDH, so long as the LDH stabilizing effect is not impaired. These other components may be included in the stabilizer or in the lactate measuring reagent (lactate measurement reagent) other than the stabilizer or may be separately added in a system when a lactate measuring reagent is used.

In one embodiment, the present disclosure provides a composition for measuring lactate or a lactate measuring reagent comprising LDH and a stabilizer to be added for improving the stability of a composition comprising LDH. In one embodiment, the lactate measuring reagent may be provided in the state of a solution or dry form. In one embodiment, a LDH stabilizer and LDH may be included as discrete reagents. In another embodiment, the compound to be added for improving the stability of a composition comprising LDH in a lactate measuring reagent, and LDH are contained in the same (single) reagent. In another embodiment, a lactate measuring reagent does not contain LDH and in this case, LDH may be added to a measurement solution at the time of measurement of lactate. In another embodiment, the lactate measuring reagent does not contain an LDH stabilizer and in this case, the stabilizer may be added to the measurement solution at the time of measurement of lactate.

In one embodiment, the present disclosure provides a method for measuring lactate by using a stabilizer to be added for improving a composition comprising LDH, and LDH. In one embodiment, the method for measuring lactate may be a method for measuring the concentration of lactate. In another embodiment, the method for measuring lactate may be a method for quantifying lactate. The sample to be measured may be a sample that comprises lactate. The stabilizer to be added for improving the stability of a composition comprising LDH may be added to a lactate measuring reagent beforehand or may be added to a measurement solution when measuring lactate.

In one embodiment, in the method for measuring lactate according to the present disclosure, a compound, which is comprised in the stabilizer to be added for improving the stability of a composition comprising LDH, can be contained in a measurement solution in a final concentration of 0.001 wt% to 50 wt%, e.g., 0.001 wt% to 25 wt%, 0.001 wt% to 20 wt%, 0.001 wt% to 15 wt%, 0.001 wt% to 10 wt%, 0.001 wt% to 9 wt%, 0.001 wt% to 8 wt%, 0.001 wt% to 7 wt%, 0.001 wt% to 6 wt%, 0.001 wt% to 5 wt%, 0.001 wt% to 4 wt%, 0.001 wt% to 3 wt%, 0.1 wt% to 2.5 wt%, 0.001 wt% to 2 wt%, 0.001 wt% to 1 wt%, 0.001 wt% to 0.5 wt%, 0.01 wt% to 10 wt%, 0.1 wt% to 10 wt%, 1 wt% to 10 wt% or 2 wt% to 10 wt%, or for example, 3 wt% to 10 wt%.

In one embodiment, in the method for measuring lactate according to the present disclosure, a compound, which is comprised in the stabilizer to be added for improving the stability of a composition comprising LDH, can be contained in a measurement solution in a final concentration of 0.1 mM to 2,000 mM, e.g., 0.1 mM to 1,500 mM, 0.1 mM to 1,000 mM, 0.1 mM to 750 mM, 0.1 mM to 500 mM, 0.1 mM to 400 mM, 0.1 mM to 300 mM, 0.1 mM to 200 mM, 0.1 mM to 100 mM, 0.1 mM to 90 mM, 0.1 mM to 80 mM, 0.1 mM to 70 mM, 0.1 mM to 60 mM, 0.1 mM to 50 mM, 0.1 mM to 40 mM, 0.1 mM to 30 mM, 0.2 mM to 20 mM, 0.25 mM to 10 mM, for example, 300 mM.

In one embodiment, in the method for measuring lactate according to the present disclosure, the stabilizer may be contained in a lactate measurement solution. In this case, measurement may be initiated by adding LDH and a sample to the lactate measurement solution. Alternatively, lactate may be measured by fixing LDH to an electrode and bringing the lactate measurement solution and a sample into contact with the electrode.

In another embodiment, the stabilizer to be added for improving stability of the composition comprising LDH of the present disclosure may be added beforehand to the lactate measuring reagent. The measuring reagent may comprise or need not comprise LDH. If the measuring reagent comprises LDH, a sample can be added to the reagent and lactate measurement can be initiated. If the measuring reagent does not comprise LDH, a sample and an LDH can be added (in any order) to the reagent and lactate measurement can be initiated. Alternatively, LDH can be fixed to an electrode and the measuring reagent and a sample can be brought into contact with the electrode to initiate measurement of lactate.

In one embodiment, a compound to be added for improving stability of the composition comprising LDH of the present disclosure does not cause precipitation of LDH. In one embodiment, ammonium sulfate at a final concentration of 50 to 70% is excluded from the compounds to be added for improving stability of the composition comprising LDH. Further, ammonium sulfate at a final concentration of 50% to 100% is excluded . If ammonium sulfate is added at a high concentration to a solution comprising LDH to cause precipitation of LDH, the supernatant thereof cannot be used for measurement of lactate.

### (LDH to which the present disclosure can be applied)

LDH to which the present disclosure can be applied, catalyzes a reaction for producing a pyruvate by oxidizing a hydroxyl group of lactate in the presence of an electron acceptor, similar to known wild-type LDHs or mutant LDHs. It should be noted that, unless specified otherwise, the substrate for LDH is lactate and the lactate may be provided as a mixture of L-form and D-form thereof, for example, a racemic form thereof, or L-form thereof.

The activity of LDH can be measured based on this principle of action by using the following measurement system employing an electron acceptor such as phenazine methosulfate (PMS) and 2,6-dichloroindophenol (DCIP), as shown in Figure 1.

(Reaction 1) L-lactate + PMS (oxidized form) → pyruvate + PMS (reduced form)

(Reaction 2) PMS (reduced form) + DCIP (oxidized form) → PMS (oxidized form) + DCIP (reduced form)

More specifically, first, in (Reaction 1), as L-lactate is oxidized, PMS (reduced form) is generated. Then, in (Reaction 2), which subsequently proceeds, as PMS (reduced form) is oxidized, DCIP is reduced. The consumption of "DCIP (oxidized form)" is detected as a change in absorbance at a wavelength of 520 nm and the activity of the enzyme can be computed based on this change in absorbance.

More specifically, the activity of LDH can be measured with the following procedure. A 1 M potassium phosphate buffer (pH 7.5) (170 µL), a 50 mM L-lactate solution (300 µL), a 1.8 mM DCIP solution (250 µL) and ultrapure water (680 µL) are mixed and kept warm at 37°C for 2 minutes or more. It should be noted that, as L-lactate, for example, L-lactate (Product No L1750) manufactured by Sigma-Aldrich can be used and in place of L-lactate, DL-lactate (e.g., Product No. 128-00056 manufactured by FUJIFILM Wako Pure Chemicals Corporation) can also be used. Then, a 30 mM PMS solution (50 µL) and an enzyme sample solution (50 µL) are added to initiate the reaction. Absorbance at 520 nm is measured when the reaction is initiated and over time and the degree of reduction (ΔA₅₂₀) in absorbance per minute is obtained with progression of the enzymatic reaction. LDH activity is obtained in accordance with the following Mathematical formula 1. In this case, LDH activity is obtained by defining the amount of the enzyme that can reduce 1 µmol DCIP per minute at 37°C in the presence of 10 mM L-lactate, as 1 U.

### [Mathematical formula 1]

It should be noted that, in the Mathematical formula, the value 1.5 represents the liquid volume (mL) of the reaction reagent + the enzyme reagent; the value 6.8 represents millimole molar absorption coefficient (mM⁻¹cm⁻¹) of DCIP in the activity measurement condition of the present disclosure; the value 0.05 represents the volume (mL) of an enzyme solution; the value 1.0 represents the optical path length (cm) of a cell; "ΔA_{520 blank}" represents the degree of reduction in absorbance at 520 nm per minute in the case where a reaction is initiated by adding a 10 mM potassium phosphate buffer (pH 6.0) comprising 0.15% (w/v) bovine serum albumin in place of an enzyme sample solution; and Df represents a dilution factor.

### (Method for evaluating thermal stability of LDH)

Thermal stability of LDH can be evaluated by heating LDH at a given temperature for a given time and comparing the activity thereof before and after heating. More specifically, a 150 mM potassium phosphate buffer (pH7.5) comprising bovine serum albumin at a final concentration of 0.15% (w/v) based on the LDH-containing buffer is placed on ice and heated separately at a given temperature (which may be e.g., 45°C, 50°C, 55°C and 60°C) for 15 minutes, and then, the LDH activity is measured. The LDH activity of an LDH solution which is allowed to stand still on ice without heating is taken as 100 and the LDH activity after heating is calculated and, the residual activity ratio (ratio of remaining activity) (%) can be determined.

### (Improvement of LDH stability)

The "improvement of stability" in the present disclosure refers not only to "improvement of the thermal stability in a solution state" but also "improvement of the thermal stability in a dry state", "improvement of the thermal stability during a drying step, "improvement of storage stability (long term stability) in a solution state" and "improvement of storage stability (long term stability) in a dry state".

More specifically, the phrase "stability is improved" in the present disclosure means that the residual activity ratio (%) of LDH, which is maintained in a composition comprising LDH after it is heated at a given temperature for a given time in the presence of a specific stabilizer or after it is stored for a long-term, is high compared to that in the absence of the stabilizer.

More specifically, the residual activity ratio (%) can be obtained by measuring LDH activity value (a) of a solution before heat treatment or before long-term storage and LDH activity value (b) of a solution after heat treatment or after long-term storage and calculating ((b)/(a) × 100). The residual activity ratio (%) of LDH when LDH is allowed to coexist with a specific candidate stabilizer compound after heat treatment or after long-term storage is calculated and this is designated as "A"; and the residual activity ratio (%) of LDH in the absence of a specific candidate stabilizer compound when LDH is subjected to the same treatment is calculated and designated as "B", and if A > B, it is evaluated that stability of LDH is improved and it is evaluated that the compound to be added in this case contributed to improvement of LDH stability.

If, for example, the residual activity ratio (%) in the absence of a stabilizer candidate compound is 10% and the residual activity ratio (%) in the presence of a stabilizer candidate compound is 15%, then it can be said that the improvement effect of a stabilizer on the stability of LDH is 5%. The improvement effect of a stabilizer on the stability of LDH may preferably be 2% or more, 3% or more, 4% or more, 5% or more, further preferably 10% or more, 15% or more, further preferably 20% or more, more preferably 30% or more, further preferably 50% or more, further preferably 60% or more, 70% or more, or 80% or more.

The improvement effect of a stabilizer on the stability of LDH can be such that when the residual activity (%) obtained in the absence of a stabilizer candidate compound is taken as 100, the relative value of the residual activity in the presence of a stabilizer candidate compound may preferably be 110 or more, further preferably 130 or more, 150 or more, 200 or more, 250 or more, 300 or more or 350 or more.

### (Origin of LDH to which the present disclosure can be applied)

The origin of LDH to which the method of the present disclosure can be applied is not particularly limited and LDH from a prokaryote, a eukaryote, a microorganism, a fungus, a plant or an animal can be used. The LDHs to which the present disclosure can be applied are flavin-dependent LDHs and the flavin compound, which serves as a coenzyme, may be flavin mononucleotide (FMN) or flavin adenine dinucleotide (FAD).

LDH from a microorganism include, but are not limited to, LDH from, for example, *Ascomycota,* preferably *Saccharomyceta,* more preferably *Saccharomycetales,* and further preferably *Saccharomycetaceae* and *Pichiaceae.* Examples of LDH from a microorganism include, but are not limited to, LDHs from yeast such as the genus *Saccharomyces,* the genus Pichia, the genus *Candida,* the genus *Ogataea,* the genus *Brettanomyces,* the genus *Cyberlindnera,* the genus *Hanseniaspora,* the genus *Kazachstania,* the genus *Kluyveromyces,* the genus *Lachancea,* the genus *Naumovozyma,* the genus *Tetrapisispora,* the genus *Torulaspora,* the genus *Vanderwaltozyma,* the genus *Zygosaccharomyces* and the genus *Zygotorulaspora.* Preferred examples of the microorganism classified in the genus *Saccharomyces* include *Saccharomyces cerevisiae, Saccharomyces pastorianus, Saccharomyces paradoxus* and *Saccharomyces eubayanus.* Preferred examples of the microorganism classified in the genus *Pichia include Pichia kudriavzevii, Pichia membranifaciens* and *Pichia capsulata.* Preferred examples of the microorganism classified in the genus Candida include *Candida inconspicua* and *Candida boidinii.* Preferred examples of the microorganism classified in the genus *Ogataea* include *Ogataea polymorpha* and *Ogataea parapolymorpha* DL-1. Preferred example of the microorganism classified in the genus *Brettanomyces* include *Brettanomyces naardenensis* and *Brettanomyces bruxellensis.* Examples of a microorganism from which LDH is derived, include, but are not limited to, microorganisms belonging to the division *Ascomycota,* the class *Sordariomycetes* e.g., the order *Sordariales,* e.g., the family, *Chaetomiaceae*)*,* e.g., the genus *Myceliophthora* or the genus *Thermothelomyces.* Example of the microorganism classified in the family *Chaetomiaceae* include, but are not limited to, microorganisms belonging to the genus *Chaetomium.* Example of the microorganism classified in the order *Sordariales* include, but are not limited to, microorganisms belonging to the genus *Madurella.* The present disclosure also encompasses combinations of domains constituting these LDHs.

It should be noted that, the classification of microorganisms is merely made for the sake of convenience. A microorganism that previously belonged to one class may sometimes be reclassified to another class. A single genus may sometimes be divided into two genera, or, two genera may sometimes be integrated into one. What is essential in the present disclosure is the microorganism that produces the LDH and not necessarily its altered academic name or classification. As such, even if academic classifications of the microorganisms mentioned above may be altered, the above disclosure should be evaluated based on the microorganism classification effective at the time of filing the present application. For example, if the family of the genus *Madurella* is not clearly determined at the time of filing the present application and if the family is taxonomically determined after the filing of the present application, provided that the family to which a microorganism is to belong, was not clearly determined at the time of filing the present application and taxonomically determined after the filing, then no matter which family the microorganism turns out to belong to, microorganisms belonging to the genus *Madurella* at the time of filing the present application are encompassed in the *Madurella* described above.

LDH, to which the method of the present disclosure can be applied, is not limited as long as the LDH has lactate dehydrogenase activity and, more specifically, the amino acid sequence of the LDH as disclosed above may further have a deletion or substitution in part of the amino acid residues or may have an addition of other amino acid residues. The present disclosure can be applied to a recombinant LDH, by obtaining a gene encoding an LDH from an LDH producing organism as disclosed above using genetic engineering methods known in the technical field and, if necessary, partially modifying the same and introducing the gene into an appropriate host microorganism and producing the recombinant LDH.

An LDH to which the present disclosure can be preferably applied is, without limitation, a flavin-dependent lactate dehydrogenase, and further preferably, a lactate dehydrogenase having the amino acid sequence of SEQ ID NO: 1, 4, 7 or 10, or lactate dehydrogenase having a sequence identity of 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, or 95% or more (e.g., 96% or more, 97% or more, 98% or more, for example, 99% or more) with the full-length amino acid sequence of SEQ ID NO: 1, 4, 7 or 10 although not limited thereto. It should be noted that, SEQ ID NOs: 1, 4, 7 and 10 are amino acid sequences after cleavage of the signal sequence. Further, the region comprising the amino acid sequence at positions 1 to 92 in SEQ ID NO: 1, a region comprising the amino acid sequence at positions 1 to 96 in SEQ ID NO: 4, a region comprising the amino acid sequence at positions 1 to 99 in SEQ ID NO: 7, and a region comprising the amino acid sequence at positions 1 to 98 in SEQ ID NO: 10 have low sequence identity to each other and it can be said that the importance of these regions is low when lactate dehydrogenase reacts with lactate. As such, lactate dehydrogenase preferably comprises, among the full-length amino acid sequence, the amino acid sequence at positions 93 to 506 in SEQ ID NO: 1, the amino acid sequence at positions 97 to 502 in SEQ ID NO: 4, the amino acid sequence at positions 100 to 505 in SEQ ID NO: 7, or the amino acid sequence at positions 99 to 503 in SEQ ID NO: 10, or an amino acid sequence having an identity of 70% or more, 75% or more, 80% or more, 85% or more, 90% or more or 95% or more with any one of the sequences mentioned above and, further, the sequence identity to a region comprising the amino acid sequence at positions 1 to 92 in SEQ ID NO: 1, a region comprising the amino acid sequence at positions 1 to 96 in SEQ ID NO: 4, a region comprising the amino acid sequence at positions 1 to 99 in SEQ ID NO: 7, and a region comprising the amino acid sequence at positions 1 to 98 in SEQ ID NO: 10 may be 45% or more, 50% or more, 60% and 70% or more or, alternatively, this region may be fully or partially deleted and, for example, 1, 2, 3, 4, 5, 6, 7, 8, 9, 10 or 15 amino acids from the N-terminal may be deleted. For example, amino acids at positions 2 to 10 in SEQ ID NO: 4 may be deleted. If a region is deleted, methionine may be appropriately added to the initiation site of the sequence. Even if the upstream part of SEQ ID NO: 4 is modified or deleted as mentioned above, the lactate dehydrogenase having such a sequence is referred to herein as a lactate dehydrogenase from *Pichia kudriavzevii.* The same applies to LDHs from other microorganisms.

LDH, in general, has a signal sequence, a cytochrome domain and a catalytic domain. The signal sequence is usually cleaved and is not involved in enzyme activity and thus, unless specified otherwise, not taken into consideration when sequence identities are compared. The cytochrome domain often has a low sequence identity, and thus, importance of the domain is low when lactate dehydrogenase reacts with lactate. Accordingly, when sequence identities are compared, unless specified otherwise, the cytochrome domain is not taken into consideration. However, this does not exclude the LDH from comprising a cytochrome domain. In one embodiment, LDH of the present disclosure has a cytochrome domain. In another embodiment, LDH of the present disclosure does not have a cytochrome domain or has only a partial of the sequence of the cytochrome domain. Further, with regard to LDHs from different origins, the present inventors confirmed that even if the cytochrome domain is exchanged, enzyme activity is exhibited. Accordingly, with regard to LDHs from the microorganism above, the catalytic domain of LDH from a first microorganism may be connected to a cytochrome domain of LDH from a second microorganism. With regard to LDHs from microorganisms, the present disclosure encompasses such combinations and fusion proteins.

### (Homologous region)

The identity or similarity of amino acid sequences can be computed with a program of, e.g., maximum matching and search homology of GENETYX Ver. 11 (manufactured by GENETYX CORPORATION) or a program of, e.g., maximum matching and multiple alignment of DNASIS Pro (manufactured by Hitachi Solutions, Ltd). An identity between amino acid sequences can be calculated by aligning the amino acid sequences of two or more LDHs and examining the positions of amino acids identical between said two or more LDHs. Based on the information, identical regions in the amino acid sequences can be determined.

Further, the positions of analogous amino acids in the amino acid sequences of two or more LDHs can also be examined. A plurality of amino acid sequences can be aligned by using, for example, CLUSTALW and, in this case, when a plurality of the amino acid sequences are aligned using Blosum62 employed as the algorithm, amino acids determined as analogous may be referred to as analogous amino acids. In mutants of the present disclosure, amino acid substitution may be a substitution between such analogous amino acids. With such alignment, it is possible to examine regions having identical amino acid sequences and positions occupied by analogous amino acids with respect to a plurality of amino acid sequences. Based on such information, a homologous region (conserved region) in an amino acid sequence can be determined.

For example, based on the lactate dehydrogenase as shown in SEQ ID NO: 4 as a reference, positions 138 to 140, positions 186 to 194, positions 217 to 222, positions 243 to 245, positions 271 to 278, positions 280 to 283, portions 355 to 367, positions 398 to 401, positions 403 to 406 and positions 425 to 433 may amount to be a homologous region. Further, the amino acid sequence of the homologous region of lactate dehydrogenase of the present disclosure may have a sequence identity of 75% or more, e.g., 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more or 98% or more or, for example, 99% or more, with the amino acid sequence of the homologous region in the sequence of SEQ ID NO: 4.

The LHD as disclosed above can be obtained artificially by protein engineering methods known in the technical field, based on information of a DNA sequence encoding the individual LHD.

As a method of using a protein engineering method, in general, a technique known as Site-Specific Mutagenesis, can be used. Examples of the technique include Kramer method (Nucleic Acids Res., 12, 9441 (1984): Methods Enzymol., 154, 350 (1987): Gene, 37, 73 (1985)), Eckstein method (Nucleic Acids Res., 13, 8749 (1985): Nucleic Acids Res., 13, 8765 (1985): Nucleic Acids Res, 14, 9679 (1986)) and Kunkel method (Proc. Natl. Acid. Sci. U.S.A., 82, 488 (1985): Methods Enzymol., 154, 367 (1987)). As a specific method for converting a nucleotide sequence in DNA, for example, use of a commercially available kit (e.g., Transformer Mutagenesis Kit manufactured by Clonetech; EXOIII/Mung Bean Deletion Kit manufactured by Stratagene; Quick Change Site Directed Mutagenesis Kit manufactured by Stratagene) can be mentioned.

The amino acid sequence of LDH may be modified artificially or randomly. If the amino acid sequence is modified artificially, a conservative amino acid substitution may be introduced into a specific site. A conservative amino acid substitution refers to substitution of an amino acid residue with another amino acid residue having a similar side chain. For example, lysine, arginine and histidine all have a basic side chain. For example, aspartic acid and glutamic acid have an acidic side chain. For example, glycine, asparagine, glutamine, serine, threonine, tyrosine and cysteine all have an uncharged polar side chain. For example, alanine, valine, leucine, isoleucine, proline, phenylalanine, methionine and tryptophan all have a non-polar side chain. For example, threonine, valine and isoleucine all have a β branched side chain. For example, tyrosine, phenylalanine, tryptophan and histidine all have an aromatic side chain. Any of these may be subjected mutually to a conservative amino acid substitution.

When an amino acid sequence is modified artificially, in specific embodiments, amino acid residues important for LDH to function, such as the active center residue of LDH, residues responsible for recognizing the substrate and residues interacting with a coenzyme, need not be substituted or are not substituted. Amino acid residues and motif sequences important for LDH to function are known in the technical field and, when the amino acid sequence of SEQ ID NO: 1 is taken as the reference, for example, tyrosine at the 139th position, tyrosine at the 249th position, histidine at the 368th position, arginine at the 371st position, alanine at the 193rd position and leucine at the 225th position can be mentioned (see, for example, Biochem., 39, 3266-3275, 2000). These positions and motif sequences need not be substituted.

Further, an artificial amino acid substitution can be used in combination with a random amino acid substitution. Further, introduction of an amino acid substitution may be carried out a plurality of times by batch units. For example, in the first round, 1 to 5 or 1 to 10 amino acid substitutions may be artificially introduced into an LDH. Then, 1 to 5 or 1 to 10 amino acid substitutions can be introduced at random to the LDH. This process can be repeated a plurality of times. The activity of the LDH mutant can be confirmed every round and, in this manner, an LDH mutant having a sequence different from the original sequence at numerous positions, for example, 10 positions or more, 20 positions or more, 30 positions or more, 40 positions or more, 50 positions or more, 60 positions or more, 70 positions or more, 80 positions or more, 90 positions or more, 100 positions or more, 110 positions or more, 120 positions or more, 130 positions or more and 140 positions or more (e.g., 150 positions or more), without loss of activity, can be prepared conveniently by repeating routine procedures. The artificial amino acid substitutions or random amino acid substitutions can be introduced by substitution into a certain region of LDH as the first set, and this can be carried out once or a plurality of times. Then, after confirming that the LDH mutant has activity, an amino acid substitution(s) may be introduced into another region of the LDH, as a second set, once or a plurality of times.

### (High-throughput screening)

LDH can be further subjected to high throughput screening in order to obtain a functional mutant. For example, a library of a transformed or transduced strains having an LDH gene having a mutation introduced thereto is prepared and subjected to high-throughput screening based on a microtiter plate or ultrahigh-throughput screening based on microfluidic droplets. For example, a combinatorial library of mutated genes encoding variants is constructed, and then, a large group of mutated LDHs is screened using e.g., phage display (e.g., Chem. Rev. 105 (11): 4056-72, 2005), yeast display (e.g., Comb Chem High Throughput Screen. 2008; 11 (2): 127-34) or bacterial display (e.g., Curr Opin Struct Biol 17: 474-80, 2007) and the like. Also see Agresti et al., "Ultrahigh-throughput screening in drop-based microfluidics for directed evolution" Proceedings of the National Academy of Sciences 107 (9): 4004-4009 (Mar, 2010). Contents of the above document with respect to ultrahigh-throughput screening methods that can be used for screening LDH variant are incorporated herein by reference. A library can be constructed by, e.g., error-prone PCR. Further, a library may be constructed by introducing a mutation targeting the positions described herein or the corresponding positions thereto using saturation mutagenesis. Using the library, appropriate cells such as electrical competent EBY-100 cells, can be transformed to obtain about ten to the power of seven mutants (10⁷). If this process is repeated 10 times, about ten to the power of eight mutants (10⁸) can be obtained. Yeast cells transformed with the library can be subsequently subjected to cell sorting. A polydimethoxyl siloxane (PDMS) microfluidic device prepared using standard soft lithography may be applied. Monodispersed droplets can be formed by a flow focus device. The formed droplets comprising individual mutants can be subjected to an appropriate sorting device. For sorting cells, the presence or absence of dehydrogenase activity can be used. Introduction of mutation and sorting can be repeated a plurality of times.

### (Obtaining LDH to which the present disclosure can be applied)

LDH to which the present disclosure can be applied can be obtained based on published literatures. For example, a microorganism producing LDH as disclosed above is cultured or a gene encoding natural LDH per se or after mutation, is inserted into an appropriate expression vector, and an appropriate host (e.g., *Escherichia coli,* yeast, *Aspergillus oryzae*) is transformed with the vector and a transformant is cultured, and then bacterial cells are collected from the culture medium by, e.g., centrifugation, and LDH is collected from the culture.

The culture medium for culturing the host cells is prepared by adding, for example, to at least one type of nitrogen source such as yeast extract, tryptone, peptone, meat extract, corn steep liquor or soy beans or a wheat bran (fusuma) exudate and the like, at least one type of inorganic salt such as sodium chloride, monopotassium phosphate, dipotassium phosphate, magnesium sulfate, magnesium chloride, ferric chloride, ferric sulfate or manganese sulfate, and, if necessary, appropriately adding a carbohydrate material and/or vitamins and the like.

The initial pH of the culture medium is not limited and can be adjusted, for example, to a pH of from 6 to 9.

Culturing may be carried out at an incubation temperature of 10 to 42°C, preferably around 25°C, for 4 to 24 hours, further preferably at an incubation temperature of around 25°C for 4 to 8 hours, in accordance with aerated/agitated submerged culture, shaking culture or static culture.

After completion of culture, LDH may be collected from the resultant culture by using an ordinary enzyme collection means known in the technical field. For example, bacterial cells can be ground by, e.g., ultrasonic disruption or grinding and the like in accordance with routine methods or, alternatively, the enzyme can be extracted using a lytic enzyme such as lysozyme and the like. Alternatively, bacterial cells can be shaken or allowed to stand still in the presence of e.g., toluene and the like, to lysis the same and, in this manner, the enzyme can be allowed to be discharged from bacterial cells. Further, this enzyme solution can be subjected to e.g., filtration or centrifugation, to remove solid matter and, if necessary, nucleic acids are removed by streptomycin sulfate, protamine sulfate or manganese sulfate and the like. Ammonium sulfate, an alcohol or acetone and the like is added to the resultant solution to fractionate the resultant solution and a precipitate is collected as a crude enzyme of LDH.

The crude enzyme of LDH can be further purified by using any means known in the technical field. A purified enzyme preparation of the present disclosure can be obtained by appropriately selecting a method from gel filtration using, for example, Sephadex, Ultrogel or Bio gel; adsorption elution using an ion exchanger; electrophoresis using e.g., polyacrylamide gel and the like; adsorption elution using hydroxyapatite; sedimentation such as sucrose density gradient centrifugation and the like; affinity chromatography; and fractionation using e.g., a molecule sieve or a hollow fiber membrane and the like or using these methods in combination. Alternatively, by using an appropriate host vector system, expressed LDH can be directly released into the culture medium.

### (Concentration of LDH)

The concentration of LDH in the present disclosure is not particularly limited. Although the appropriate range of the concentration varies depending on the characteristics and the like of the enzyme (LDH), the LDH can be at any concentration as long as those skilled in the art can determine that lactate can be measured with sufficient reliability using said enzyme at that concentration.

Although the concentration of LDH in the present disclosure is not particularly limited, for example, the concentration of LDH in a solution is preferably, 0.01 to 1,000 U/mL, further preferably, 0.1 to 100 U/mL and further preferably 0.1 to 10 U/mL. While LDH is preferably present in a similar concentration in a powder or a lyophilizate, for preparing a powder preparation, a concentration of 100 U/mL or more can be employed.

The composition comprising LDH of the present disclosure can be provided in liquid state and also can be converted into a powder product by e.g., lyophilization, vacuum drying or spray drying. Although the composition of a buffer to be used in extraction, purification, and powderization of LDH and in stability tests, is not particularly limited, the composition of the buffir may be any composition so long as it has buffer capacity within the pH range of preferably 4 to 9. Examples of the buffer include commercially available buffers such as boric acid, tris-HCL, and potassium phosphate and the like, and commercially available good's buffers such as BES, Bicine, Bis-Tris, CHES, EPPS, HEPES, HEPPSO, MES, MOPS, MOPSO, PIPES, POPSO, TAPS, TAPSO, TES and Tricine. These buffers may be used alone or in combination with two or more.

Although the concentration of a buffer is not particularly limited as long as it has the necessary buffer capacity, the upper limit is preferably 100 mM or less and more preferably 50 mM or less. The lower limit is preferably 5 mM or more. Although the content of a buffer in a powder or a lyophilizate is not particularly limited, the concentration to be used preferably falls within the range of 0.1% (weight ratio) or more and particularly preferably 0.1 to 30% (weight ratio).

In one embodiment, the present disclosure provides a method for screening a stabilizer that improves stability of LDH. This method may comprise the steps of:
i) providing an LDH,
ii) applying a heat treatment to the LDH at 35 to 70°C for 5 to 60 minutes,
iii) after the step ii), determining the residual activity of the LDH,
iv) applying a heat treatment to the LDH in the presence of a candidate substance instead of applying the treatment to the LDH alone, in step ii), followed by determining the residual activity of the LDH, and
v) comparing the residual activity obtained in step iii) and the residual activity obtained in the presence of a candidate substance in step iv). If the residual activity obtained in the presence of a candidate substance in step iv) is higher than the residual activity obtained in step iii), the candidate substance can be taken as a stabilizer that improves the stability of LDH.

### (Production of a composition comprising LDH)

A composition with improved LDH stability can be produced by allowing LDH to coexist with any one of the stabilizers as disclosed above for improving the stability of LDH. The composition comprising the enzyme of the present disclosure is not limited to a lyophilized product and, the composition may be in the state of a solution obtained by re-dissolving a dried product. More specifically, in the composition of the present disclosure, LDH may be allowed to coexist with any one of the stabilizers by any method. For example, if both substances are provided in a solution state, a composition can be produced by mixing them. If both substances are provided in a powder form, these substances can be mixed to produce a composition. When LDH is powderized and then granulated, a stabilizer can be added during the granulation step. Further, when a plurality of stabilizers are used, some of the stabilizers may be added in a step of producing LDH, the other remaining stabilizers can be added in a step of preparing a lactate measuring reagent or a step of fixing the same to a lactate sensor.

In one embodiment, the present disclosure provides a method for producing a composition comprising LDH. The method may comprise the steps of;
i) providing an LDH,
ii) applying a heat treatment to the LDH at 35 to 70°C for 5 to 60 minutes,
iii) after the step ii), determining the residual activity of the LDH,
iv) applying a heat treatment to the LDH in the presence of a candidate substance instead of applying the treatment to the LDH alone, in step ii), followed by determining the residual activity of the LDH,
v) comparing the residual activity obtained in step iii) and the residual activity obtained in the presence of a candidate substance in step iv), and
vi) producing a composition with improved LDH stability by allowing LDH to coexist with a candidate substance if the residual activity in the presence of the candidate substance in step iv) is higher than that obtained in step iii).

In one embodiment, the present disclosure provides a method for suppressing the decrease or deactivation of lactate dehydrogenase activity in a method of producing an electrode comprising a step of applying lactate dehydrogenase to an electrode. The method may comprise the steps of:
i) providing a solution comprising lactate dehydrogenase,
ii) adding, to the solution, at least one stabilizer selected from the group consisting of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; polyethylene glycol; and an ionic polymer,
iii) applying the solution comprising the lactate dehydrogenase and the stabilizer to an electrode, and
iv) drying the applied solution.

In one embodiment, the electrode prepared by applying lactate dehydrogenase can be included in a lactate sensor.

### (Mediator)

In one embodiment, a mediator known in the technical field can be used when LDH is used being fixed or without being fixed to an electrode. Examples of the mediator include phenazine compounds such as 1-methoxy-5-methylphenazinium methyl sulfate (mPMS), 5-methylphenazinium methyl sulfate (PMS) and 5-ethylphenazinium methyl sulfate (PES) and the like; ferricyanides such as phenothiazine compounds and potassium ferricyanide; ferrocene compounds such as ferrocene, dimethylferrocene and ferrocene carboxylic acid and the like; quinone compounds such as naphthoquinone, anthraquinone, hydroquinone and pyrroloquinoline quinone and the like; cytochrome compounds; viologen compounds such as benzyl viologen and methyl viologen and the like; indophenol compounds such as dichlorophenol indophenol and the like; ruthenium complexes such as hexaammineruthenium chloride and the like; osmium complexes such as osmium-2,2'-bipyridine and the like; and phenylenediamine compounds such as p-phenylenediamine and N-isopropyl-N'-phenyl-p-phenylenediamine and the like.

These mediators may be modified with a polymer such as polyvinylimidazole, polyethylene imine and polyacrylic acid and used. These mediators can be fixed to LDH by using, e.g., a cross-linking reagent and the like. The entire contents are incorporated herein by reference. These mediators can be fixed to an electrode after the surface of the electrode is activated by treating the same with, e.g., an acid. As the material for the electrode, e.g., carbon, gold and platinum can be used.

The final concentration of a mediator to be added to a sample solution is not particularly limited and may fall within the range of, for example, 1 pM to 1 M, 1 pM to 100 mM, 1 pM to 20 mM, 1 pM to 10 mM, 1 pM to 5 mM, 2 pM to 1 mM, 3 pM to 800 µM, 4 pM to 600 µM, 5 pM to 500 µM, 6 pM to 400 µM, 7 pM to 300 µM, 8 pM to 200 µM, 9 pM to 100 µM or 10 pM to 50 µM. It should be noted that, the order of adding a mediator and other reagents is not limited and they may be added simultaneously or sequentially.

### (Reagent for measuring lactate, lactate assay kit, lactate sensor)

A reagent for measuring lactate, a lactate assay kit and a lactate sensor with improved LDH stability can be produced by using the composition comprising LDH of the present disclosure, and lactate can be measured by using any one of the same.

The reagent for measuring lactate of the present disclosure typically comprises reagents required for measurement, i.e., LDH, a buffer and a mediator and the like; a lactate standard solution for preparing a calibration curve with instructions, and comprises a stabilizer that contributes to improving the stability of LDH.

The reagent for measuring lactate of the present disclosure can be provided, for example, as a lyophilized product or as a solution in an appropriate storage solution. The reagent for measuring lactate of the present disclosure can be used to measure lactate in the same manner as in a conventional reagent for measuring lactate. The reagent for measuring lactate of the present disclosure exhibits more excellent thermal stability and storage stability when used because the stability of LDH is improved

### (Lactate assay kit)

The lactate assay kit of the present disclosure typically comprises a sufficient amount of LDH required for at least a single-time lactate measurement, a buffer required for assay, a mediator, a lactate standard solution for preparing a calibration curve and a stabilizer contributing to improving the stability of LDH. The lactate assay kit of the present disclosure can be used in the same manner as in a conventional lactate assay kit to measure lactate. The lactate assay kit of the present disclosure exhibits more excellent thermal stability and storage stability when used because the stability of LDH is improved.

### (Lactate sensor)

In one embodiment, the present disclosure provides a lactate sensor with improved thermal stability of LDH. In one embodiment, the sensor comprises an electrode. In one embodiment, the sensor comprises a working electrode and a counter electrode as the electrode. In one embodiment, the sensor comprises a working electrode, a counter electrode and a reference electrode, as the electrode. In one embodiment, LDH is fixed to an electrode. As the working electrode, a carbon electrode, a gold electrode or a platinum electrode can be used and, the enzyme of the present disclosure can be fixed on the electrode. Examples of a fixation method include a method of using a cross-linking reagent, a method of embedding the enzyme in a polymer matrix, a method of coating the enzyme with a dialysis membrane, a method of using a photocrosslinkable polymer, a conductive polymer or a redox polymer, a method of fixing the enzyme together with a mediator in a polymer and a method of fixing the enzyme onto the electrode by adsorption. Further, these fixation methods may be used in combination. As a typical method, LDH of the present disclosure is fixed onto a carbon electrode with glutaraldehyde and treated with a reagent having an amine group to block the glutaraldehyde. A composition or reagent comprising the stabilizer of the present disclosure can be used for various electrochemical measurements in the form of e.g., an electrode onto which LDH is fixed or an enzyme sensor, in combination with, e.g., a potentiostat or a galvanostat. Examples of the electrochemical measurement method include various methods such as amperometry, e.g., chronoamperometry; potential step chronoamperometry; voltammetry e.g., cyclic voltammetry; differential pulse voltammetry, potentiometry and coulometry and the like. For example, with regard to lactate measurement, measuring the current value when lactate is reduced by using amperometry, the concentration of lactate in a sample can be calculated. The voltage to be applied varies depending on the conditions and setting of a device, and can be set at, for example, -1,000 mV to +1,000 mV (vs. Ag/AgCl).

As the electrode, a printed electrode can be used. This enables reducing the volume of the solution required for measurement. The electrode can be formed on an insulating substrate. More specifically, the electrode can be formed on a substrate by a printing technique such as photolithography, screen printing, gravure printing or flexo printing. Examples of the material for an insulating substrate include silicon, glass, ceramic, polyvinyl chloride, polyethylene, polypropylene, and polyester, and materials having high resistance against various solvents and chemical agents can be used. Examples of the base for an electrode include carbon cloth, carbon paper and bucky paper. The area of the working electrode can be set in accordance with a desired response current. For example, in one embodiment, the area of the working electrode may be set at, for example, 1 mm² or more, 1.5 mm² or more, 2 mm² or more, 2.5 mm² or more, 3 mm² or more, 4 mm² or more, 5 mm² or more, 6 mm² or more, 7 mm² or more, 8 mm² or more, 9 mm² or more, 10 mm² or more, 12 mm² or more, 15 mm² or more, 20 mm² or more, 30 mm² or more, 40 mm² or more, 50 mm² or more, 1 cm² or more, 2 cm² or more, 3 cm² or more, 4 cm² or more or 5 cm² or more, e.g., 10 cm² or more. In one embodiment, the area of the working electrode may be set at 10 cm² or less, or 5 cm² or less (e.g., 1 cm² or less). The same applies to the counter electrode. Further, the apparent surface area of the working electrode can be increased by fixing, e.g., carbon nanotube, graphene or Ketjen black onto the electrode. In this case, the apparent area can be increased 10 times or more, 50 times or more, 100 times or more or 1000 times or more.

The concentration of lactate can be measured by chronoamperometry, for example, as follows. A buffer and a mediator are added into a thermostatic cell, which is kept at a constant temperature. An electrode onto which LDH of the present disclosure is fixed is used as a working electrode, and a counter electrode (e.g., platinum electrode) and a reference electrode (e.g., Ag/AgCl electrode) are used. A constant voltage is applied to the carbon electrode and, after a constant current is obtained, a sample comprising lactate is added to measure (an increase of) the current. According to a calibration curve prepared by using a standard-concentration lactate solution, the concentration of lactate in a sample can be calculated.

The lactate sensor of the present disclosure can measure lactate in the same manner as in a conventional lactate sensor. When the lactate sensor of the present disclosure is used, the lactate sensor of the present disclosure exhibits more excellent thermal stability and storage stability since stability of LDH is improved. Further, in the step of fixing LDH to the sensor, the degree of deactivation due to heat at the time of the fixation can be reduced and, therefore, the amount of LDH to be used in fixation can be reduced.

### (Anode electrode of the present disclosure)

In one embodiment, the present disclosure provides an anode electrode comprising an LDH composition with improved thermal stability. In one embodiment, LDH may be fixed onto an electrode of a battery. In one embodiment, a battery can be provided by using the anode electrode of the present disclosure in combination with a cathode electrode and a resistance. In one embodiment, the present disclosure provides a method for generating electricity using the battery. In another embodiment, the present disclosure provides a battery comprising an LDH composition with improved thermal stability.

### (Fuel cell of the present disclosure)

In one embodiment, the present disclosure provides an anode or a cathode for a fuel cell and a fuel cell comprising such anode or the cathode. The fuel cell comprises an LDH composition with improved thermal stability according to the present disclosure. In one embodiment, the present disclosure provides a method for generating electricity using a battery comprising the LDH composition with improved thermal stability and a method for generating electricity by fixing LDH to an anode electrode using a substrate for LDH, for example, lactate, as a fuel, and generating electricity.

In one embodiment, the fuel cell of the present disclosure has an anode comprising an LDH composition with improved thermal stability, a fuel vessel, a cathode and an electrolyte. An artificial electronic mediator may be allowed to be adsorbed onto the anode or cathode. Examples of the artificial electronic mediator include, but are not limited to, mediators known in the technical field mentioned described and phenylenediamine compounds disclosed in JP Patent No. 6484741 and JP Patent No. 6484742. In the fuel cell of the present disclosure, if necessary, a load resistor may be placed between the anode and cathode and wiring can be provided to arrange the resistor. In one embodiment, the load resistor is a part of the fuel cell of the present disclosure. In one embodiment, the load resistor is not a part of the fuel cell of the present disclosure, and the fuel cell of the present disclosure is constituted such that it can be connected to an appropriate load resistor. In the fuel cell of the present disclosure, an oxidoreductase (LDH) constitutes part of the anode. For example, an oxidoreductase may be placed in proximity or in contact with of the anode or may be fixed or adsorbed onto the anode. The fuel vessel contains a compound serving as a substrate for the oxidoreductase fixed to the electrode. In one embodiment, the fuel cell of the present disclosure may have an ion exchange membrane, which separates the anode and the cathode. The ion exchange membrane may have pores of 1 nm to 20 nm. The anode may be a general electrode such as a carbon electrode. For example, an electrode formed of a conductive carbonaceous material such as carbon black, graphite, activated carbon and an electrode formed of a metal such as gold or platinum, can be used. Examples of the conductive carbonaceous material include carbon paper, carbon cloth, glassy carbon and HOPG (highly oriented pyrolytic graphite). As the cathode used in pair, platinum or platinum alloy and the like, an electrode in which an electrocatalyst generally used in fuel cells is allowed to be carried by a carbonaceous material such as carbon black, graphite and activated carbon, or a conductive material consisting of, e.g., gold or platinum, can be used and, further, a conductive material consisting of an electrocatalyst such as platinum or a platinum alloy, itself, can be used as the cathode electrode and, an oxidant (e.g., substrate for a cathode, oxygen) may be supplied to the electrocatalyst.

In another embodiment, as a cathode to be used in pair with an anode consisting of a substrate-oxidizing enzyme electrode, a substrate-reducing enzyme electrode can be used. Examples of an oxidoreductase that reduces an oxidizing agent include enzymes known in the technical field such as laccase and bilirubin oxidase and the like. If an oxidoreductase is used as a catalyst for reducing an oxidant, if necessary, an electron transfer mediator known in the technical field may be used. Examples of the oxidant include oxygen and the like.

In one embodiment, to avoid effects due to impurities (e.g., ascorbic acid and uric acid and the like), which interrupt the electrode reaction at a cathode, an oxygen-selective membrane (e.g., dimethylpolysiloxane membrane) can be placed around the cathode.

The method for generating electricity of the present disclosure includes a step of supplying a compound serving as a fuel, which is a substrate for an oxidoreductase, to an anode comprising the oxidoreductase. When the fuel is supplied to the anode comprising the oxidoreductase, the substrate is oxidized and simultaneously an electron is generated, and the electron is transferred by the oxidoreductase to an electron transfer mediator that mediates transfer of an electron between the oxidase and the electrode, such as a phenylenediamine compound, and then, is transferred to a conductive substrate (anode electrode) by the electron transfer mediator. When the electrons migrate through the wiring (external circuit) from the anode electrode to a cathode electrode, a current is generated.

The protons (H⁺), which are generated in the process above, migrate through the electrolyte solution to the cathode electrode. Them, in the cathode electrode, protons that have migrated from the anode in the electrolyte solution and electrons that have migrated from the anode side through an external circuit react with an oxidant (cathode-side substrate) such as oxygen or hydrogen peroxide, to generate water. Electricity can be generated by utilizing this.

The present disclosure will be more specifically described hereby by way of Examples. However, the technical scope of the present disclosure is not limited by Examples in any way.

### Examples

### 1. Preparation of plasmid for expressing LDH

### Preparation of recombinant plasmids

As disclosed in Biochem. J. 258, 255-259 (1989), a gene of 1521 bp (including stop codon TAA) as shown in SEQ ID NO: 2 and encoding 506 amino acids as shown in SEQ ID NO: 1, which is obtained by removing amino acids at positions 1 to 85 from 591 the amino acids constituting the amino acid sequence of lactate dehydrogenase from *Saccharomyces cereviciae* (ScLDH), was obtained as a double strand DNA, by a routine method, i.e., PCR of a gene fragment. Into a multicloning site of plasmid pKK223-3, the target gene, i.e., ScLDH gene, was inserted in accordance with routine methods and a DNA construct was prepared. More specifically, in the In-Fusion Cloning Site present in the multiple cloning site of pKK223-3, the ScLDH gene was ligated using In-Fusion HD Cloning Kit (manufactured by Clontech Laboratories, Inc.) in accordance with the protocol attached to the kit to obtain an expression plasmid (pKK223-3-ScLDH). Further, *Escherichia coli* JM109 was transformed with this plasmid to obtain *Escherichia coli* JM109 (pKK223-3-ScLDH) strain, and this was inoculated to 3 mL of LB-amp medium [1% (w/v) Bacto-tryptone, 0.5% (w/v) peptone, 0.5% (w/v) NaCl, 50 µg/mL ampicillin] and cultured at 37°C for 16 hours while shaking and a culture was obtained. The culture was centrifuged at 10,000 × g for one minute and bacterial cells were collected. From the bacterial cells, recombinant plasmid pKK223-3-ScLDH was obtained using GenElute Plasmid Miniprep Kit (manufactured by Sigma-Aldrich).

Subsequently, a gene of 1509 bp (including stop codon TAA) as shown in SEQ ID NO: 5 and encoding the 502 amino acids as shown in SEQ ID NO: 4, which is obtained by removing amino acids at positions 2 to 77 from 578 amino acids as shown in SEQ ID NO: 3 constituting the amino acid sequence of lactate dehydrogenase from *Pichia kudriavzevii* (PkLDH), as compared to ScLDH, was obtained as a double strand DNA by a routine method, i.e., PCR of a gene fragment. In the same manner, a gene of 1518 bp (including stop codon TAA) as shown in SEQ ID NO: 8 and encoding 505 amino acids and as shown in SEQ ID NO: 7, which is obtained by removing amino acids at positions 2 to 67 from 571 amino acids as shown in SEQ ID NO: 6 constituting the amino acid sequence of lactate dehydrogenase from *Candida inconspicua* (CaLDH), was obtained as a double strand DNA by a routine method, i.e., PCR of a gene fragment. In the same manner, a gene of 1512 bp (including stop codon TAA) as shown in SEQ ID NO: 11 and encoding the 503 amino acids and as shown in SEQ ID NO: 10, which is obtained by removing amino acids at positions 2 to 56 from 558 amino acids as shown in SEQ ID NO: 9 constituting the amino acid sequence of lactate dehydrogenase from *Ogataea parapolymorpha* (OgLDH) was obtained as a double strand DNA by a routine method, i.e., PCR of a gene fragment. These three types of genes, were inserted into the multicloning site of plasmid pKK223-3 in the same manner as in the preparation of the ScLDH expression plasmid to obtain recombinant plasmids pKK223-3-PkLDH, pKK223-3-CaLDH and pKK223-3-OgLDH, respectively.

### 2. Purification of LDH preparations

As an LDH-producing bacterium, the strain *Escherichia coli* BL21 was used. First, *Escherichia coli* BL21 (pKK223-3-ScLDH) strain transduced with pKK223-3-ScLDH was cultured in advance in LB medium (comprising 100 µg/mL ampicillin) on a plate and a colony of the strain on the medium was picked with a toothpick and inoculated to a small test tube containing 2 mL of LB medium (comprising 100 µg/mL ampicillin) and cultured at 25°C and 160 rpm for 24 hours while shaking. The total amount of the medium was transferred to a 0.5 L Sakaguchi flask containing 250 mL of LB medium (comprising 50 µg/mL ampicillin and 1 mM isopropyl-β-thiogalactopyranoside (hereinafter referred to as IPTG)) and cultured at 25°C and 130 rpm for 30 hours while shaking. In the same manner, *Escherichia coli* BL21 (pKK223-3-PkLDH) strain transduced with pKK223-3-PkLDH was cultured in a small test tube containing 2 mL of LB medium (comprising 100 µg/mL ampicillin) at 30°C and 160 rpm, overnight, while shaking. The total amount of the medium was transferred to a 0.5 L Sakaguchi flask containing 250 mL of LB medium (comprising 50 µg/mL ampicillin and 1 mM IPTG) and cultured at 37°C and 130 rpm for 30 hours while shaking. In the same manner, *Escherichia coli* BL21 (pKK223-3-CaLDH) strain and *Escherichia coli* BL21 (pKK223-3-OgLDH) strain transduced with pKK223-3-CaLDH and pKK223-3-OgLDH, respectively, were cultured in small test tubes containing 2 mL of LB medium (comprising 100 µg/mL ampicillin) at 30°C and 160 rpm, overnight, while shaking. The total amount of the medium was transferred to a 0.5 L Sakaguchi flask containing 250 mL of LB medium (comprising 50 µg/mL ampicillin and 1 mM IPTG) and cultured at 30°C and 130 rpm for 30 hours while shaking.

After completion of culture, the culture solution was centrifuged at 6,000 rpm and 25°C for 5 minutes and bacterial cells were collected by removing the supernatant. Subsequently, the obtained bacterial cells were suspended in a 20 mM potassium phosphate buffer (pH7.5).

The bacterial cell suspension was ultrasonically crushed using an ultrasonic homogenizer US-150E (manufactured by Nippon Seiki Co., Ltd.) until a translucent suspension was obtained, and the suspension was centrifuged at 9,000 rpm and 4°C for 10 minutes and the supernatant was recovered. The supernatants of crushed bacterial cells comprising various types of LDHs except ScLDH were purified by anion exchange chromatography. More specifically, first, each of the supernatants comprising various types of LDHs was loaded onto 10 mL of a resin, i.e., Q Sepharose Fast Flow (manufactured by Cytiva) equilibrated with a 20 mM potassium phosphate buffer (pH 7.5). Subsequently, a 20 mM potassium phosphate buffer (pH 7.5) comprising 0 to 500 mM NaCl was added and fractions exhibiting LDH activity were recovered. The fractions exhibiting LDH activity were concentrated using Amicon (registered trademark) Ultra-15 Centrifugal Filters Ultracel (registered trademark)-30K. Subsequently, the concentrated fractions were allowed to be adsorbed onto Hiscreen Capto Q ImpRes column (manufactured by Cytiva), and in the case of PkLDH, fractions exhibiting LDH activity were recovered with a gradient of 250 mM to 450 mM NaCl/20 mM potassium phosphate buffer (pH 7.0). In purification of CaLDH and OgLDH, fractions exhibiting LDH activity were recovered by a gradient of 150 mM to 350 mM NaCl/20 mM potassium phosphate buffer (pH 7.0). Subsequently, the recovered fractions were each subjected to HiLoad 26/600 Superdex 200 pg column (manufactured by Cytiva) equilibrated with a 10 mM potassium phosphate buffer (pH 7.0 in the case of PkLDH, pH 7.5 in the cases of CaLDH and OgLDH) comprising 150 mM sodium chloride. The fractions obtained were analyzed by SDS-PAGE and fractions giving a single band and exhibiting LDH activity were recovered and used as purified preparations of the LDHs.

### 4. Verification 1 of LDH stability improving effects due to addition of various compounds

Using the purified PkLDH preparation obtained above, the residual activity ratio (%) in the presence and absence of an LDH stabilizer were compared in accordance with the LDH thermal stability evaluation method mentioned previously, and in this manner, a stabilizer was searched.

The thermal stability of LDH was evaluated by heating LDH at a given temperature for a given time and comparing the activities before and after heating. More specifically, a 150 mM potassium phosphate buffer (pH7.5) comprising 0.15% (w/v) bovine serum albumin (final concentration in the buffer comprising LDH) was allowed to stand still on ice and heated individually at a given temperature (which may e.g., be 45°C, 50°C, 55°C, 60°C) for 15 minutes, and thereafter, LDH activities were measured. Further, LDH activity of an LDH solution allowed to stand still on ice without any heat treatment was measured. Based on the activity of this case without any heat treatment taken as 100, the activities of LDH solutions after heat treatment were calculated to determine the residual activity ratio (%). More specifically, the residual activity ratio (%) can be obtained by measuring LDH activity value (a) of a solution before heat treatment or before long-term storage, and LDH activity value (b) of a solution after heat treatment or after long-term storage, and then, calculating ((b)/(a) × 100).

The degree of LDH stability improving effect of each of the various added compounds was evaluated by preparing an LDH solution by allowing LDH to coexist with each of the various compounds, subjecting the solution to a heat treatment and determining the residual activity ratio (%) of the solution, and evaluating the residual activity (%) by mutually comparing the cases between compounds or comparing the case to a case where no compound was added.

The activity of LDH was determined in accordance with the following procedure. A 1 M potassium phosphate buffer (pH7.5) (170 µL), a 50 mM DL-lactate (manufactured by FUJIFILM Wako Pure Chemicals Corporation, Product No. 128-00056) solution (300 µL), a 1.8 mM DCIP solution (250 µL) and ultrapure water (680 µL) were mixed and kept warm at 37°C for 2 minutes or more. Subsequently, a 30 mM PMS solution (50 µL) and an enzyme sample solution (50 µL) were added to the mixture to initiate a reaction. Absorbance at 520 nm was measured at the time of initiation of the reaction and over time and, the degree of reduction (ΔA₅₂₀) in absorbance per minute as the enzymatic reaction progresses was obtained and LDH activity was calculated in accordance with the following Mathematical formula 2. Here, LDH activity is expressed by an enzyme (LDH) unit, and 1U is defined as the amount of the enzyme that can reduce 1 µmol DCIP per minute at 37°C in the presence of 10 mM L-lactate.

### [Mathematical formula 2]

It should be noted that, in the Mathematical formula, the value 1.5 represents the liquid volume (mL) of the reaction reagent + the enzyme reagent; the value 6.8 represents the millimole molar absorption coefficient (mM⁻¹cm⁻¹) of DCIP in the activity measurement condition of the present disclosure; the value 0.05 represents the volume (mL) of the enzyme solution; the value 1.0 represents the optical path length (cm) of the cell; "ΔA_{520 blank}" represents the amount of reduction in absorbance at 520 nm per minute in the case where a reaction is initiated by adding a 10 mM potassium phosphate buffer (pH 6.0) comprising 0.15% (w/v) bovine serum albumin in place of an enzyme sample solution; and Df represents a dilution factor.

In accordance with the evaluation method for thermal stability disclosed above, the residual activity ratio of LDH was calculated using a 150 mM potassium phosphate buffer and an LDH solution (2 U/mL) comprising each of the compounds at 300 mM and 0.15% (w/v) bovine serum albumin (BSA). The pH of the PkLDH solution was adjusted to be 7.5 after each of the compounds was added. Heat treatment was carried out at 55°C for 15 minutes. As a comparative example, an LDH solution prepared without adding any compound was subjected to the same experiment. It should be noted that, unless specified otherwise, the reagents used herein were all purchased from Tokyo Chemical Industry Co., Ltd.

As a result of these experiments, it was found that thermal stability of LDH is improved by adding any one of sodium sulfate, ammonium sulfate, trehalose dihydrate, sucrose, D-(-)-tartaric acid, L-malic acid, sodium citrate, sodium gluconate, succinic acid, maleic acid, fumaric acid, L-sodium aspartate, L-glutamic acid and L-arginine hydrochloride, as shown in Table 1. The LDH stability improvement effects of these stabilizers were 10% or more, and it was confirmed that they have a high stability improving effect.

In particular, in the cases where ammonium sulfate, L-malic acid, sodium gluconate, succinic acid, L-sodium aspartate, L-glutamic acid and L-arginine hydrochloride were added, for PkLDH, the residual activity ratio (%) was 50% or more, whereas the residual activity ratio (%) of for the case without adding any stabilizers was 30%. That is, the improvement effects of these stabilizers on the stability of LDH were 20% or more, and it was confirmed that these compounds have a high stability improvement effect. In particular, when L-malic acid was added, the residual activity ratio (%) was 78%. That is, the stability of LDH was greatly improved by L-malic acid, and it was found that L-malic acid has a significantly high stability improvement effect.

**[Table 1]**

| Added compound (300 mM) | Residual activity ratio (%) |
|---|---|
| None (comparative example) | 30 |
| Sodium sulfate | 50 |
| Ammonium sulfate | 54 |
| Trehalose dihydrate | 41 |
| Sucrose | 42 |
| L-malic acid | 78 |
| Sodium citrate | 42 |
| Sodium gluconate | 57 |
| Succinic acid | 54 |
| Maleic acid | 45 |
| Fumaric acid | 41 |
| Sodium aspartate | 64 |
| L-glutamic acid | 58 |
| L-arginine hydrochloride | 63 |

### 5. Verification 2 of LDH stability improving effects due to addition of malic acid analog compounds

The stability evaluation test was carried out with compounds having structures analogous to that of L-malic acid in accordance with the method disclosed in Section 4. A comparative example was carried out using a PkLDH solution (comprising 150 mM potassium phosphate buffer (pH7.5) and 0.15% (w/v) BSA) prepared without adding any compound, in the same manner as above. Examples of the L-malic acid analogue compounds to be added include D-malic acid, DL-leucine acid, DL-3-hydroxyaspartic acid, DL-3-phenyllactic acid, oxaloacetic acid, D-(-)-tartaric acid and L-(+)-tartaric acid, as shown in Table 2.

As a result of these experiments, it was found that the stability of LDH is improved by adding any one of D-malic acid, DL-3-hydroxyaspartic acid, oxaloacetic acid, D-(-)-tartaric acid and L-(+)-tartaric acid, as shown in Table 2. In particular, in the cases where DL-3-hydroxyaspartic acid, D-(-)-tartaric acid and L-(+)-tartaric acid were added, the residual activity ratio (%) was above 50%, whereas the residual activity ratio (%) of the case without adding any compound was 30%. It was confirmed that these compounds have a high stability improvement effect. Among these, DL-3-hydroxyaspartic acid, DL-leucine acid and DL-phenyllactic acid exhibited a residual LDH activity ratio (%) of 77%, 92% and 87%, respectively. That is, it was found that addition of any one of DL-3-hydroxyaspartic acid, DL-leucine acid and DL-phenyllactic acid produces a significantly high improvement effect on LDH stability.

**[Table 2]**

| Added compound (300 mM) | Residual activity ratio (%) |
|---|---|
| None | 30 |
| L-malic acid | 79 |
| D-malic acid | 47 |
| DL-3-hydroxyaspartic acid | 77 |
| DL-leucine acid | 92 |
| DL-phenyllactic acid | 87 |
| Oxaloacetic acid | 45 |
| D-(-)-tartaric acid | 60 |
| L-(+)-tartaric acid | 52 |

### 6. Verification 3 of LDH stability improving effects due to addition of malic acid

Stability evaluation tests were conducted to check the improvement effect of malic acid on the stability of various LDHs. LDH preparations (PkLDH, CaLDH, OgLDH) obtained above were each diluted with an enzyme diluent (prepared by adding L-malic acid in 150 mM in potassium phosphate buffer so as to obtain a final concentration of 300 mM, adjusting pH of the buffer to be pH 7.5 with NaOH, and thereafter, adding bovine serum albumin so as to obtain a final concentration of 0.15% (w/v)) so as to obtain an enzyme concentration of 2 U/mL. Each of these diluted enzyme solutions (a mixture of an enzyme and L-malic acid, LDH concentration: 2 U/mL, L-malic acid concentration: 300 mM) was heated in accordance with the thermal-stability evaluation method described in Section 4. More specifically, PkLDH was treated at 55°C, whereas, CaLDH and OgLDH were treated at 50°C for 15 minutes and then the residual activity ratio (%) was calculated, based on the activity before heating taken as 100. As a comparative example, a sample without adding any L-malic acid in the enzyme diluent was subjected to the same experiment, and the ratio of residual activity in both cases were compared.

As a result, it was confirmed that the stabilities of all three types of LDHs, are improved by addition of L-malic acid, as shown in Table 3.

**Table 3**

| Added compound (300 mM) | Residual activity ratio (%) | | |
|---|---|---|---|
| | PkLDH | CaLDH | OgLDH |
| None | 30 | 2 | 14 |
| L-malic acid | 80 | 45 | 88 |

### 7. Verification 4 of LDH stability improving effects due to addition of malic acid

Concentration dependency of the stabilizing effect of malic acid addition was examined. The LDH preparation (PkLDH) obtained above was diluted with an enzyme diluent (prepared by adding L-malic acid in 150 mM in a potassium phosphate buffer so as to obtain a final concentration of 10 mM, 30 mM, 100 mM, 300 mM and 500 mM, adjusting pH of the buffer to be pH 7.5 with NaOH, and thereafter, adding bovine serum albumin so as to obtain a final concentration of 0.15% (w/v)) so as to obtain a solution with an enzyme concentration of 2 U/mL. The diluted enzyme solutions (a mixture of an enzyme (LDH) and L-malic acid, LDH concentration: 2 U/mL, L-malic acid concentration: 10 mM, 30 mM, 100 mM, 300 mM, 500 mM) were subjected to a heat treatment performed at 55°C for 15 min in accordance with the method described in Section 4. After the heat treatment, the residual activity ratio (%) of each of the diluted solutions was calculated based on the activity thereof before heating taken as 100.

As a result, the tendency was confirmed that the stability of LDH is improved by increasing the concentration of malic acid, as shown in Table 4.

**[Table 4]**

| Final concentration of malic acid (mM) | Residual activity ratio (%) |
|---|---|
| 0 | 30 |
| 10 | 36 |
| 30 | 44 |
| 100 | 52 |
| 300 | 80 |
| 500 | 90 |

### 8. Verification 2 of LDH stability improving effects due to addition of various compounds

Using the purified CaLDH preparation obtained above, the ratios of remaining activities (%) in the presence and absence of a LDH stabilizer were compared in accordance with the LDH thermal stability evaluation method described in Section 4. In this manner, a stabilizer was explored.

The residual activity ratio was calculated by using an LDH solution (2 U/mL) comprising a 150 mM potassium phosphate buffer, each of 300 mM compounds (maltose monohydrate, ethylenediaminetetraacetic acid) and 0.15% (w/v) bovine serum albumin (BSA). The pH of the LDH solution was adjusted to be 7.5 after each of the compounds was added. Heat treatment was carried out at 50°C for 15 minutes. As a comparative example, an LDH solution (comprising 150 mM potassium phosphate buffer (pH was adjusted to 7.5) and 0.15% (w/v) BSA) was prepared without adding any compound and subjected to the same experiment.

As a result, the improvement effect of maltose monohydrate on the stability of LDH was 5%, whereas, the improvement effect of ethylenediaminetetraacetic acid on the stability of LDH was 56%. That is, it was found that the thermal stability of LDH is improved by adding either one of maltose monohydrate and ethylenediaminetetraacetic acid.

### 9. Verification 3 of LDH stability improving effects due to addition of various compounds

Using the purified OgLDH preparation obtained above, the ratios of remaining activities (%) in the presence and absence of a LDH stabilizer were compared in accordance with the LDH thermal stability evaluation method described in Section 4. In this manner, a stabilizer was searched.

The residual activity ratio of LDH was calculated by using an LDH solution (2 U/mL) comprising a 150 mM potassium phosphate buffer, each of 300 mM compounds (glycine, L-lysine hydrochloride, L-histidine, disodium malonate) and 0.15% (w/v) BSA. The pH of the LDH solution was adjusted to be 7.5 after each of the compounds was added. Heat treatment was carried out at 50°C for 15 minutes. As a comparative example, an LDH solution (comprising 150 mM potassium phosphate buffer (pH was adjusted to 7.5) and 0.15% (w/v) BSA) was prepared without adding any compound and subjected to the same experiment.

As a result, the LDH stability improving effect of glycine was 11%; the LDH stability improving effect of L-lysine hydrochloride was 66%; the LDH stability improving effect of L-histidine was 27%; and the LDH stability improving effect of disodium malonate was 96%. That is, it was found that thermal stability of LDH is improved by adding any one of glycine, L-lysine hydrochloride, L-histidine and disodium malonate. Among the same, disodium malonate, in particular, produced the highest improvement effect on the stability of LDH.

### 10. Verification 4 of LDH stability improving effects due to addition of various compounds

Using the supernatant of a solution comprising crushed ScLDH bacterial cells obtained above, the ratios of remaining activities (%) in the presence and absence of a LDH stabilizer were compared in accordance with the LDH thermal stability evaluation method described in Section 4. In this manner, a stabilizer was searched.

The residual activity ratio of LDH was calculated by using an LDH solution (2 U/mL) comprising a 150 mM potassium phosphate buffer, each of 300 mM compounds (L-malic acid, disodium malonate and ammonium sulfate) and 0.15% (w/v) BSA. The pH of the LDH solution was adjusted to be 7.5 after each of the compounds was added. Heat treatment was carried out at 40°C for 15 minutes. As a comparative example, an LDH solution (comprising 150 mM potassium phosphate buffer (pH was adjusted to 7.5) and 0.15% (w/v) BSA) was prepared without adding any compound and subjected to the same experiment.

As a result, the stability improving effect of L-malic acid on ScLDH was 41%; the stability improving effect of disodium malonate on ScLDH was 26%; and the stability improving effect of ammonium sulfate on LDH was 24%. That is, it was found that thermal stability of ScLDH is improved by adding any one of L-malic acid, disodium malonate and ammonium sulfate, similarly to PkLDH, CaLDH and OgLDH.

### 11. Verification 5 of LDH stability improving effects due to addition of various compounds

Using the purified PkLDH preparation obtained above, a stabilizer was searched in the same procedure as in the LDH thermal stability evaluation method described in Section 4 except that 150 mM HEPES buffer was used in place of the potassium phosphate buffer. The residual activity ratio of LDH was calculated by using each of the 300 mM compounds listed in Table 5 and an LDH solution (2 U/mL) comprising 0.15% (w/v) bovine serum albumin (BSA) in accordance with the thermal stability evaluation method disclosed above. It should be noted that, only for the cases of polyethylene glycol and polylysine, these were added to obtain a final concentration of 2%. The pH of the LDH solution was adjusted to be 7.5 after each of the compounds was added. Heat treatment was carried out at 55°C for 15 minutes. As a comparative example, an LDH solution (comprising 150 mM HEPES buffer (pH was adjusted to 7.5) and 0.15% (w/v) BSA) was prepared without adding any compound and subjected to the same experiment.

As a result, it was found that the LDH stability was improved by adding any one of potassium phosphate, polyethylene glycol and ε-polylysine (Table 5). In particular, when each of potassium phosphate and polylysine was added, the residual activity ratio (%) exceeds 70% while the residual activity ratio (%) of the case where no compound was added was 27%, and it was confirmed that a significantly high stability was confirmed in these cases. Further, stabilizers such as L-malic acid and disodium malonate found so far exerted stabilizing effects not only when they are added to a phosphate buffer but also to a HEPES buffer, and were thus found to be effective as a stabilizer regardless of the type of buffer. This indicates that various compounds contributing to stability improvement can exert an effect regardless of the type of buffer.

**[Table 5]**

| Added compound | Residual activity ratio (%) |
|---|---|
| None | 27 |
| 300 mM potassium phosphate | 76 |
| 300 mM L-malic acid | 100 |
| 350 mM disodium malonate | 101 |
| 2% polyethyleneglycol | 38 |
| 2% ε polylysine (MW: 3500-4500) | 73 |

### 12. Verification 6 of LDH stability improving effects due to addition of various compounds

Using the purified PkLDH preparation obtained above, a stabilizer was searched in a similar manner in accordance with the LDH thermal stability evaluation method described in Section 4 above. Using an LDH solution (3 U/mL) comprising each of the compounds (2% (w/v)) listed in Table 6 and 0.15% (w/v) bovine serum albumin (BSA), the residual activity ratio of LDH was calculated in accordance with the thermal stability evaluation method disclosed above. The pH of each of the LDH solutions was adjusted to be 7.5 after each of the compounds was added. Heat treatment was carried out at 55°C for 15 minutes. As a comparative example, an LDH solution (comprising 150 mM potassium phosphate buffer (pH was adjusted to 7.5) and 0.15% (w/v) BSA) was prepared without adding any compound and subjected to the same experiment.

The degree of LDH stability improving effect of each of the various added stabilizers was evaluated by determining the residual activity ratio of LDH (%) of a sample comprising a stabilizer and comparing with the residual activity ratio (%) of a sample without adding anystabilizer. More specifically, a relative value of the residual activity ratio of a sample comprising a stabilizer to the residual activity ratio (taken as 100) of a sample without adding any stabilizer was calculated. If the relative value is larger than 100, the sample was evaluated to have a stability improvement effect. For example, if a comparative sample without adding any stabilizer has a residual activity ratio of 30% and a sample to which a stabilizer was added has a residual activity ratio of 45%, the relative value of the residual activity is 150.

As a result of the experiments, it was found that LDH stability is improved when α-polylysine and each of 2 types of polyethylene imines having different average molecular weight and side-chain structure were added (Table 6). In particular, when a branched polyethylene imine was added, a relative value of the residual activity to that (100) of the case without adding any compound exceeded 290 and it was confirmed that significantly high stability is provided by the branched polyethylene imine. Further, in the case of a cationic polymer, such as polylysine and polyethylene imine, a stabilizing effect was exerted regardless of the molecular weight, and even if the side chain of a cationic polymer differs, the polymer was found to be effective as a stabilizer. From this, it is indicated that the polymer compounds contributing to stabilization improvement can exert an effect regardless of the molecular weight.

**[Table 6]**

| Added compound | Relative value of residual activity |
|---|---|
| None | 100 |
| 2% α polylysine (MW: 1000-5000) | 152 |
| 2% polyethylene imine (straight chain, MW: about 4000) | 256 |
| 2% polyethylene imine (branched chain, MW: about 10000) | 296 |

### 13. Verification 7 of LDH stability improving effects due to addition of various compounds

Using the purified PkLDH preparation obtained above, a stabilizer was searched in the same procedure as in the LDH thermal stability evaluation method described in Section 4. Using an LDH solution (3 U/mL) comprising each of the compounds having the mass % and molar concentration disclosed in Table 7 and 0.15% (w/v) bovine serum albumin (BSA), the ratio of the remaining LDH activity was calculated in accordance with the thermal stability evaluation method disclosed above. The pH of each of the LDH solutions was adjusted to be 7.5 after each of the compounds was added. Heat treatment was carried out at 55°C for 15 minutes. As a comparative example, an LDH solution (comprising 150 mM potassium phosphate buffer (pH was adjusted to 7.5) and 0.15% (w/v) BSA) was prepared without adding any compound and subjected to the same experiment.

As a result, it was found that LDH stability is improved when any one of sodium hyaluronate, methyl glycol chitosan, carboxymethylcellulose sodium and poly(diallyldimethylammonium chloride) was added (Table 7). A polymer that can serve as a stabilizer may have any structure as long as a side chain has an anionic group such as a carboxy group or a cationic group such as an amino group as a functional group, and it is indicated that such polymer exhibits a stabilizing effect regardless of its structure.

**[Table 7]**

| Added compound | Relative value of residual activity |
|---|---|
| None | 100 |
| 0.1% sodium hyaluronate | 123 |
| 0.2% carboxymethylcellulose sodium | 123 |
| 5 mM methyl glycol chitosan | 179 |
| 0.2% poly(diallyldimethylammonium chloride) | 170 |

### 14. Verification 1 of the effect of addition of the stabilizer based on current measurement using a printed electrode to which LDH was applied

An enzyme electrode was prepared using the purified PkLDH preparation obtained above and chronoamperometry was carried out. Each of the compounds was added to an enzyme solution in advance and tested whether or not a stabilizing effect of the enzyme during a drying step was exerted. LDH (1 U) solutions comprising compounds in a concentration (0.2% (w/v), 2% (w/v) and 50 mM, respectively) as described in Table 8 were each applied to a screen-printed carbon electrode C110 (manufactured by Metrohm-Dropsens) in an amount of 4 µL and dried at 40°C for 60 minutes. The same procedure was repeated using an LDH solution without adding any one of the compounds. Each of the electrodes prepared was connected to ALS electrochemical analyzer 814D (manufactured by BAS) using a dedicated connector (DRP-CAC, manufactured by Drop Sense). Subsequently, 100 µL of a 2 mM mPMS/PBS solution was added dropwise to dissolve the dried LDH. The applied voltage was +200 mV (vs Ag/Ag+). An L-sodium lactate solution was added and a response current value 5 seconds after initiation of measurement was recorded. As a result, the current value was large in all cases comprising a compound compared to the case in which no compound was added, as shown in Table 8. Further, in the same manner as above, a 1 U LDH solution comprising 1 % polyacrylic acid was applied to a screen printed carbon electrode and dried and then a 2 mM mPMS/PBS solution (100 µL) was added dropwise to dissolve the dried LDH. Then, an L-sodium lactate solution was added such that the final concentration thereof would be 2 to 10 mM, and a response current value 5 seconds after initiation of measurement was recorded. As a result, the current value was 20 µA when L-sodium lactate (a final concentration of 10 mM) was added. The current value was 14 µA when polyacrylic acid was not added, and the current value was higher when polyacrylic acid was added. Further, the current values when L-sodium lactate was added within a final concentration of 2 to 10 mM were plotted on the vertical axis and lactate concentrations were plotted on the horizontal axis to make a graph, and the linearity of the relationship between the current value and the lactate concentration was R² = 0.97, and it was demonstrated that L-lactate can be quantified. From the results, it was indicated that a compound that produced an effect in the state of a solution could suppress deactivation of the enzyme during a drying step for preparing an electrode and exerts an enzyme stabilizing effect.

**[Table 8]**

| Added compound | Current value 5 seconds later (µA) | |
|---|---|---|
| | 0 mM lactate | 2 mM lactate |
| None | 0.06 | 13.8 |
| 50 mM sodium malonate | 0.21 | 61.8 |
| 2% ε polylysine | 0.10 | 33.4 |
| 0.2% polyethylene imine | 0.41 | 38.5 |

### 15. Verification 2 of the effect of addition of the stabilizer based on current measurement using a printed electrode to which LDH was applied

Chronoamperometry was carried out by changing the concentration of the compound to be added as a stabilizer and the concentration of L-sodium lactate used as a substrate. To an electrode prepared in the same manner as in the preceding Section, 100 µL of a 5 mM mPMS/PBS solution was added dropwise to dissolve the dried LDH. A voltage of +200 mV (vs Ag/Ag+) was applied. An L-sodium lactate solution was added and a response current value 5 seconds after initiation of measurement was recorded. As a result, the current value was larger in the cases where sodium malate and myo-inositol were added compared to the case of not adding any compound, as shown in Table 9. Even if the concentration of the added polymer was reduced, the current value was larger compared to the case of not adding any compound. Furthermore, compounds that exhibited a stability improvement effect in the above experiment also produced a stability improvement effect within the range of a lactate concentration of 0 to 10 mM in this experiment. From the results, it was found that a compound that produced an effect in the state of a solution can suppress deactivation of the enzyme during the drying step for preparing an electrode and produces an enzyme stabilizing effect, regardless of its concentration. It was also found that when LDH reacts on the electrode with lactate within the range of 0 to 10 mM, even if the added compound coexisted, LDH sufficiently exerts activity.

**[Table 9]**

| Added compound | Current value 5 second later (µA) | |
|---|---|---|
| | 0 mM lactate | 5 mM lactate |
| None | 0.19 | 49.9 |
| 50 mM L-sodium malate | 0.43 | 94.4 |
| 50 mM myo-inositol | 0.23 | 104.4 |
| 2% polyethylene imine | 0.24 | 98.4 |
| 0.2% polyethylene imine | 0.34 | 141.1 |
| 0.02% polyethylene imine | 0.34 | 121.0 |
| 2% ε polylysine | 0.33 | 106.3 |
| 0.2% ε polylysine | 0.34 | 112.8 |
| 0.02% ε polylysine | 0.41 | 133.0 |
| 0.2% sodium hyaluronate | 0.26 | 98.0 |
| 0.02% sodium hyaluronate | 0.25 | 84.5 |

It should be noted that, although not shown in the table, it was also confirmed that an improvement effect on the stability of LDH is exerted when 10 mM lactate is present, as in the case of 5 mM lactate.

All publications, patents and patent applications cited in the present specification are incorporated herein in their entireties.

### Explanation of sequences

SEQ ID NO: 1 lactate dehydrogenase from *Saccharomyces cereviciae* (ScLDH) (amino acids at positions 1 to 85 in the wild type ScLDH are removed)
SEQ ID NO: 2 Nucleotide sequence encoding the amino acid sequence of SEQ ID NO: 1
SEQ ID NO: 3 Amino acid sequence of lactate dehydrogenase from *Pichia kudriavzevii* (PkLDH)
SEQ ID NO: 4 Sequence obtained by removing the sequence at positions 2 to 77 from SEQ ID NO: 3
SEQ ID NO: 5 Nucleotide sequence encoding the amino acid sequence of SEQ ID NO: 4
SEQ ID NO: 6 Amino acid sequence of lactate dehydrogenase from *Candida inconspicua* (CaLDH)
SEQ ID NO: 7 Sequence obtained by removing the sequence at positions 2 to 67 from SEQ ID NO: 6
SEQ ID NO: 8 Nucleotide sequence encoding the amino acid sequence of SEQ ID NO: 7
SEQ ID NO: 9 Amino acid sequence of lactate dehydrogenase from *Ogataea parapolymorpha* (OgLDH)
SEQ ID NO: 10 Sequence obtained by removing the sequence at positions 2 to 56 from SEQ ID NO: 9
SEQ ID NO: 11 Nucleotide sequence encoding the amino acid sequence of SEQ ID NO: 10

## Claims

1. A method for improving thermal stability of a composition comprising lactate dehydrogenase, said method comprising a step of subjecting a composition comprising lactate dehydrogenase requiring a flavin compound as a coenzyme to coexist with at least one of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; a polyethylene glycol; or an ionic polymer.

2. The method according to claim 1, wherein the lactate dehydrogenase is a lactate dehydrogenase from the genus *Saccharomyces,* the genus *Pichia,* the genus *Candida,* the genus *Ogataea,* the genus *Brettanomyces,* the genus *Cyberlindnera,* the genus *Hanseniaspora,* the genus *Kazachstania,* the genus *Kluyveromyces,* the genus *Lachancea,* the genus *Naumovozyma,* the genus *Tetrapisispora,* the genus *Torulaspora,* the genus *Vanderwaltozyma*, the genus *Zygosaccharomyces,* the genus *Zygotorulaspora*, the genus *Myceliophthora,* the genus *Thermothelomyces*, the genus *Chaetomium or* the genus *Madurella.*

3. The method for improving thermal stability of a composition comprising lactate dehydrogenase according to claim 1, wherein the lactate dehydrogenase is the following (i) and/or (ii):
(i) lactate dehydrogenase comprising an amino acid sequence, which has an identity of 70% or more or 80% or more to the amino acid sequence of SEQ ID NO: 1, SEQ ID NO: 4, SEQ ID NO: 7 or SEQ ID NO: 10,
(ii) lactate dehydrogenase, **characterized in that** the amino acid sequence of a homologous region consisting of amino acid sequences at positions 138 to 140, positions 186 to 194, positions 217 to 222, positions 243 to 245, positions 271 to 278, positions 280 to 283, positions 355 to 367, positions 398 to 401, positions 403 to 406 and positions 425 to 433 in SEQ ID NO: 4 has a sequence identity of 80% or more or 90% or more to the amino acid sequence of a homologous region of the corresponding positions in the lactate dehydrogenase.

4. The method for improving thermal stability of a composition comprising lactate dehydrogenase according to any one of claims 1 to 3, wherein
the monocarboxylic acid is selected from the group consisting of gluconic acid, leucine acid, 3-phenyllactic acid, glycine, lysine, histidine and arginine,
the dicarboxylic acid is selected from the group consisting of malic acid, succinic acid, maleic acid, fumaric acid, malonic acid, methylmalonic acid, oxaloacetic acid, tartaric acid, oxalic acid, succinic acid, glutaric acid, α-ketoglutaric acid, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid and 3-hydroxyasparagine,
the tricarboxylic acid is selected from the group consisting of citric acid, isocitric acid, aconitic acid, trimesic acid, 1,2,3-propanetricarboxylic acid and 2-phosphonobutane-1,2,4-tricarboxylic acid,
the tetracarboxylic acid is selected from ethylenediaminetetraacetic acid,
the phosphoric acid is selected from the group consisting of a phosphate and a pyrophosphate,
the sulfuric acid is selected from a sulfate,
the monosaccharide is selected from the group consisting of myo-inositol,
the disaccharide is selected from the group consisting of sucrose, trehalose and maltose, or
polyethylene glycol is used,
the ionic polymer is an anionic polymer or a cationic polymer,
the anionic polymer is selected from the group consisting of a polyacrylic acid, hyaluronic acid and carboxymethyl cellulose or a salt thereof, or
the cationic polymer is selected from polylysine, polyethylene imine, methyl glycol chitosan and poly(diallyldimethylammonium chloride).

5. The method for improving thermal stability of a composition comprising lactate dehydrogenase according to any one of claims 1 to 4, wherein the anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; an anionic polymer; or a cationic polymer is included in a solution within a concentration range of from 0.01 wt% to 30 wt%.

6. A composition for measuring lactate comprising: a lactate dehydrogenase requiring a flavin compound as a coenzyme; and at least one compound of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; polyethylene glycol; and an ionic polymer.

7. The composition for measuring lactate according to claim 6, wherein the lactate dehydrogenase is a lactate dehydrogenase from the genus *Saccharomyces,* the genus *Pichia,* the genus *Candida,* the genus *Ogataea,* the genus *Brettanomyces,* the genus *Cyberlindnera*, the genus *Hanseniaspora*, the genus *Kazachstania*, the genus *Kluyveromyces,* the genus *Lachancea*, the genus *Naumovozyma*, the genus *Tetrapisispora*, the genus *Torulaspora,* the genus *Vanderwaltozyma,* the genus *Zygosaccharomyces,* the genus *Zygotorulaspora*, the genus *Myceliophthora,* the genus *Thermothelomyces*, the genus *Chaetomium or* the genus *Madurella.*

8. The composition according to claim 6, wherein the lactate dehydrogenase is the following (i) and/or (ii),
(i) lactate dehydrogenase comprising an amino acid sequence, which has an identity of 70% or more or 80% or more to the amino acid sequence of SEQ ID NO: 1, SEQ ID NO: 4, SEQ ID NO: 7 or SEQ ID NO: 10,
(ii) lactate dehydrogenase, **characterized in that** the amino acid sequence of a homologous region consisting of amino acid sequences at positions 138 to 140, positions 186 to 194, positions 217 to 222, positions 243 to 245, positions 271 to 278, positions 280 to 283, positions 355 to 367, positions 398 to 401, positions 403 to 406 and positions 425 to 433 in SEQ ID NO: 4 has a sequence identity of 80% or more or 90% or more to the amino acid sequence of a homologous region of the corresponding positions in the lactate dehydrogenase.

9. The composition for measuring lactate according to any one of claims 6 to 8, wherein
the monocarboxylic acid is selected from the group consisting of gluconic acid, leucine acid, 3-phenyllactic acid, glycine, lysine, histidine and arginine,
the dicarboxylic acid is selected from the group consisting of malic acid, succinic acid, maleic acid, fumaric acid, malonic acid, methylmalonic acid, oxaloacetic acid, tartaric acid, oxalic acid, succinic acid, glutaric acid, α-ketoglutarate, adipic acid, pimelic acid, suberic acid, azelaic acid, sebacic acid, phthalic acid, isophthalic acid, terephthalic acid, glutaconic acid, tartronic acid, muconic acid, mesaconic acid, citraconic acid, meconic acid, 3-3'-dimethylglutaric acid, itaconic acid, glutamic acid, aspartic acid and 3-hydroxyasparagine,
the tricarboxylic acid is selected from the group consisting of citric acid, isocitric acid, aconitic acid, trimesic acid, 1,2,3-propanetricarboxylic acid and 2-phosphonobutane-1,2,4-tricarboxylic acid,
the tetracarboxylic acid is selected from ethylenediaminetetraacetic acid,
the phosphoric acid is selected from the group consisting of a phosphate and a pyrophosphate,
the sulfuric acid is selected from a sulfate,
the monosaccharide is selected from the group consisting of myo-inositol,
the disaccharide is selected from the group consisting of sucrose, trehalose and maltose, or
polyethylene glycol is used,
the ionic polymer is an anionic polymer or a cationic polymer,
the anionic polymer is selected from the group consisting of a polyacrylic acid, hyaluronic acid and carboxymethyl cellulose or a salt thereof, or
the cationic polymer is selected from polylysine, polyethylene imine, methyl glycol chitosan and poly(diallyldimethylammonium chloride).

10. The composition for measuring lactate according to any one of claims 6 to 9, wherein the anion selected from the group consisting of a monocarboxylic acid, a dicarboxylic acid, a tricarboxylic acid, a tetracarboxylic acid, a polycarboxylic acid, phosphoric acid, sulfuric acid or a salt thereof (excluding ammonium sulfate); a monosaccharide; a disaccharide; a polyethylene glycol; an anionic polymer; or a cationic polymer is included in a solution within a concentration range of from 0.01 wt% to 30 wt%.

11. A kit for measuring lactate comprising the compound according to any one of claims 6 to 10.

12. An electrode comprising a composition comprising lactate dehydrogenase requiring a flavin compound as a coenzyme and at least one compound selected from the group consisting of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; polyethylene glycol; and an ionic polymer.

13. A lactate sensor comprising the electrode according to claim 12.

14. A fuel cell comprising the electrode according to claim 12.

15. A method of suppressing the decrease or deactivation of lactate dehydrogenase activity in a method of producing an electrode comprising a step of applying lactate dehydrogenase to an electrode, said method comprising the steps of:
i) providing a solution comprising lactate dehydrogenase,
ii) adding, to the solution, at least one stabilizer selected from the group consisting of an anion selected from the group consisting of a monocarboxylic acid, a dicarboxylic acid, a tricarboxylic acid, a tetracarboxylic acid, a polycarboxylic acid, phosphoric acid, sulfuric acid or a salt thereof; a monosaccharide; a disaccharide; polyethylene glycol; and an ionic polymer,
iii) applying the solution comprising lactate dehydrogenase and the stabilizer to an electrode, and
iv) drying the solution applied.

16. The production method according to claim 15, wherein the electrode to which lactate dehydrogenase is applied is comprised in a lactate sensor.
